(19) Europäisches Patentamt

European Patent Office

Office européen des brevets

(11) **EP 0 662 000 B1**

(12) **FASCICULE DE BREVET EUROPEEN**

(45) Date de publication et mention de la délivrance du brevet:
**12.07.2000 Bulletin 2000/28**

(51) Int. Cl.⁷: **A61K 39/385**, A61K 47/48, C07K 17/08

(21) Numéro de dépôt: **93919446.0**

(22) Date de dépôt: **13.09.1993**

(86) Numéro de dépôt international:
**PCT/FR93/00876**

(87) Numéro de publication internationale:
**WO 94/06472 (31.03.1994 Gazette 1994/08)**

(54) **MICROPARTICULES PORTANT DES ANTIGENES ET LEUR UTILISATION POUR L'INDUCTION DE REPONSES HUMORALES OU CELLULAIRES**

ANTIGEN ENTHALTENDE MIKROPARTIKEL UND IHRE VERWENDUNG FÜR DIE INDUZIERUNG VON HUMORALEN ODER ZELLULÄREN ANTWORTEN

ANTIGEN-CARRYING MICROPARTICLES AND THEIR USE IN THE INDUCTION OF HUMORAL OR CELLULAR RESPONSES

(84) Etats contractants désignés:
**AT BE CH DE DK ES FR GB GR IE IT LI LU MC NL PT SE**

(30) Priorité: **11.09.1992 FR 9210879**

(43) Date de publication de la demande:
**12.07.1995 Bulletin 1995/28**

(73) Titulaire: **INSTITUT PASTEUR 75724 Paris Cédex 15 (FR)**

(72) Inventeurs:
• **GENGOUX, Christine**
  **F-95100 Argenteuil (FR)**
• **LECLERC, Claude**
  **F-75015 Paris (FR)**

(74) Mandataire: **Phélip, Bruno**
  **c/o Cabinet Harlé & Phélip**
  **7, rue de Madrid**
  **75008 Paris (FR)**

(56) Documents cités:
  **EP-A- 0 465 081       FR-A- 2 304 326**

• JOURNAL OF IMMUNOLOGICAL METHODS vol. 52, no. 3 , 1982 pages 341 - 351 A. REMBAUM ET AL. 'CELL LABELING AND MAGNETIC SEPARATION BY MEANS OF IMMUNOREAGENTS BASED ON POLYACROLEIN MICROSPHERES' cité dans la demande
• EUROPEAN JOURNAL OF IMMUNOLOGY vol. 17 , Septembre 1987 pages 1287 - 1296 H. KIRK ZIEGLER ET AL. 'DIFFERENTIAL REQUIREMENTS FOR THE PROCESSING AND PRESENTATION OF SOLUBLE AND PARTICULATE BACTERIAL ANTIGENS BY MACROPHAGES.' cité dans la demande

**Description**

La présente invention a pour objet des microparticules portant en surface des antigènes et leur utilisation pour l'induction de réponses humorales ou cellulaires.

Plus spécifiquement, l'invention concerne également des microparticules comportant, en surface, une densité importante d'antigènes.

Les cellules B qui expriment des récepteurs immunoglobulines spécifiques pour un antigène particulier sont hautement efficaces pour la présentation de cet antigène. (Rock et al. C., J. Exp. Med. (1984) 160; 1102; Hutchings et al. Eur. J. Immunol. (1987) 17:393). Par exemple, des cellules B spécifiques peuvent présenter la toxoïde tétanique à des cellules T à des concentrations on antigène $IO^4$ fois inférieures à celles requises pour la présentation par des cellules B non spécifiques ou des monocytes du sang périphérique. (Lanzavecchia, Nature, (1985) 314:537).

De plus, des études in vivo avec des souris déficientes en cellules B indiquent que ces cellules sont requises pour l'activation des cellules T des ganglions lymphatiques (Janeway et al. J. Immunol. (1987) 138:1051; Ron, et al. J., J. Immunol. (1987) 138:2848; Kurt-Jones et al. A.K. J. Immunol. (1987) 140:3773).

Les souris déficientes en cellules B présentent aussi des réponses réduites en ce qui concerne les cellules T $CD4^+$ et $CD8^+$ spécifiques de tumeurs, après immunisation par le virus de la leucémie murine de Freund (Schultz et al., Science, (1990) 291).

La capacité des cellules B à modifier et à présenter l'antigène en vue de la reconnaissance par des cellules T Helper $CD4^+$ restreintes au complexe majeur d'histocompatibilité de classe II (MHC) forme la base d'un modèle d'activation des cellules B par les cellules T. (Noëlle et al. The Faseb Journal. (1991) 5:2770).

La reconnaissance par des cellules T Helper $CD4^+$ du complexe peptide-MHC de classe II à la surface des cellules B permet la formation de conjugués stables physiquement entre les cellules T et les cellules B (Kupfer et al. S. J.. Proc. National Acad. Sci. USA. (1986) 83:6O8O).

Cette reconnaissance directe a pour résultat la prolifération et la différenciation de cellules B en réponse à des lymphokines telles l'Interleukine-2, l'Interleukine-4 ou l'Interleukine-5.

L'induction de la réponse anticorps contre un antigène nécessite donc la présentation de l'antigène par des cellules B.

La plupart des études sur la présentation de l'antigène ont été effectuées en utilisant des protéines solubles telles que la toxoïde du tétanos, le lysozyme, l'hémocyanine (LH). Cependant, la plupart des antigènes auxquels le système immunitaire est exposé, sont inclus dans des structures particulaires complexes telles que des bactéries ou des parasites.

Il est bien établi que les cellules qui sont capables de phagocytose telles que les macrophages peuvent présenter des antigènes bactériens à des cellules T.

Par contre, on ne sait pas si des cellules qui ne phagocytent pas, telles que les cellules B, peuvent présenter des antigènes complexes et de tailles importantes.

Il a été montré récemment que, in vivo, des antigènes bactériens devaient être mis sous une forme soluble, pour induire une réponse anticorps dépendante des cellules T (Leclerc et al. J. Immunol. (1990) 144:3174; Leclerc et al. J. Immunol. (1991) 147:3545).

Il convenait cependant d'établir encore qu'in vivo, les antigènes protéiques bactériens sont exclusivement présentés aux cellules T par des cellules phagocytaires et que les cellules B ne peuvent modifier des antigènes sous forme de particules.

A cet effet, on a comparé, selon la présente invention, la capacité de macrophages et de cellules B à présenter le même antigène, sous une forme soluble et particulaire.

On a notamment utilisé des antigènes protéiques, tels que le lysozyme et le TNP-KLH, couplés à des microparticules de poly(acroléine) ou de polystyrène ayant une taille comparable à celle d'une bactérie.

Selon l'invention, on a montré de manière surprenante que les cellules B qui présentent le TNP-KLH ou le lysozyme de manière très efficace, sont incapables de présenter ces antigènes couplés à des billes. Par contre, les macrophages présentent les deux formes d'antigènes aux cellules T.

L'étude de la présentation des antigènes et de l'induction de la réponse T cellulaire et/ou humorale a une importance scientifique et médicale particulière.

En effet, l'orientation vers une réponse purement cellulaire ou une réponse purement humorale peut permettre de vacciner contre certains pathogènes, de modifier certains dysfonctionnements biologiques et de guérir certaines pathologies.

Par exemple, une telle orientation permettrait d'éliminer des infections persistantes ou d'opérer une régulation des réponses allergiques.

De plus, il existe deux sous-populations de cellules T, $CD4^+$, les Th1 et les Th2 qui ont des capacités différentes à produire diverses lymphokines (Mosmann, Cherwinski, Bond, Giedlin et Coffman, J. Immunol., 136, 2348-2357 (1986)). L'induction de Th1 ou de Th2 joue un rôle majeur dans la résistance aux infections bactériennes, parasitaires ou virales. Ainsi, dans le cas de la leishmaniose cutanée murine, les Th1 protègent de l'infection alors que les Th2 aggravent la maladie. In vitro, les lymphocytes B stimulent de façon optimale la prolifération des clones Th2 alors qu'une forte prolifération des clones Th1 est observée avec les cellules adhérentes (Gajewski, Pinnas, Wong et Fitch. J. Immunol., 146, 1750-1758 (1991)).

L'orientation de l'antigène vers la présentation par des cellules B ou des macrophages peut permettre d'induire des réponses Th1 ou Th2.

Différentes techniques ont été développées jusqu'à présent pour induire une meilleure réponse immunitaire.

La plus ancienne des méthodes consiste à activer le système immunitaire à l'aide d'adjuvants. Ainsi, l'adjuvant de Freund permet d'augmenter l'intensité des réponses humorale et cellulaire. Cependant, de tels adjuvants présentent des inconvénients majeurs dus à leur manque de spécificité, à leur toxicité et aux réactions immunologiques parasites qu'ils risquent d'induire, en raison de leur manque de pureté.

Les iscomes (abréviation de l'expression immuno-stimulating complexes) sont composés d'un complexe antigénique et d'un adjuvant, le QuilA qui est extrait d'un arbre. Ces particules ont un diamètre d'environ 35 nm et sont composées de sous-unités d'environ 12nm. Elles permettent d'induire une réponse immunologique mais le plus souvent les antigènes sont encapsulés et sont donc ensuite relargués dans le milieu extérieur. En outre, la technique ne permet pas un contrôle précis du type de cellules présentant ces particules, et de ce fait ces particules induisent une double réponse humorale et cellulaire.

Enfin, du point de vue pratique, on notera des difficultés de préparation, un manque de stabilité et une toxicité importante de ces particules.

Les liposomes dont l'utilisation a aussi été testée pour l'induction d'une réponse immunitaire présentent les mêmes inconvénients que les iscomes.

Des microparticules biodégradables comme par exemple des polymères d'acide lactique et glutamique ont aussi été développées (Aguado et Lambert, Immuno. Biol., 184, 113-125, (1992)). Ces microparticules libèrent au cours de leur dégradation, l'antigène sous forme soluble. Cette libération permet une présentation de l'antigène par diverses cellules et l'induction d'une réponse humorale sans possibilité d'orientation vers une réponse spécifiquement cellulaire.

Des particules constituées exclusivement de protéines recombinantes ont aussi été synthétisées. Ainsi, la demande de brevet français FR 2.635.532 décrit des particules composées d'une protéine hybride entre l'antigène HBs et une séquence immunogène supposée induire des anticorps neutralisants dirigés contre des virus HIV.

Ces particules présentent des inconvénients notables. Ainsi, il est très difficile d'insérer des séquences longues dans ces particules. De plus, elles induisent tant une réponse humorale que cellulaire et il n'est donc pas possible d'obtenir spécifiquement l'une des deux.

Des particules de polyacroléine ou de polystyrène auxquelles sont couplés des anticorps ont déjà été utilisées pour la mise au point de techniques de séparation (Rembaum et al., Immunol. (1982) 52:341-351).

Aucune utilisation pour la préparation de vaccins et l'immunisation in vivo n'est néanmoins mentionnée. Les billes utilisées ont des diamètres de 20 à 35 nm (polyacroléine) ou de 40 à 120 µm (polystyrène).

Des particules de polyacroléine d'un diamètre de 2 µm ont aussi été utilisées pour étudier in vitro la stimulation des réponses T (Ziegler et al. Eur. J. Immunol. (1987), 17: 1287-1296). L'activité de ces billes n'est pas testée in vivo.

Dans l'ensemble de ces travaux, le critère de la taille des particules n'a jamais été considéré comme critique. Cependant, des particules de petites tailles (nanoparticules) comme les particules HBs pourraient être présentées par les lymphocytes B. Au contraire, des particules de taille trop importante (supérieures à 5-10 microns) ne peuvent pas être présentées par des cellules phagocytaires.

Les diverses solutions proposées dans l'état de la technique d'une part pour permettre une induction d'une réponse immunologique importante et d'autre part pour diriger cette réponse spécifiquement dans une des deux voies de réponse, humorale ou cellulaire, ne sont donc pas satisfaisantes.

L'invention propose de mettre au point des produits permettant d'obtenir une bonne réponse immunologique tout en ayant une orientation cellulaire ou humorale.

Selon l'invention, on a trouvé de manière surprenante qu'une telle réponse pouvait être induite à l'aide de microparticules, de faibles tailles et présentant des densités antigéniques variées.

La présente invention a particulièrement pour objet des microparticules en matériau synthétique polymère portant en surface une ou plusieurs protéines ou peptides liés de manière covalente au matériau constituant les microparticules, la ou lesdits protéines ou peptides portant chacun un ou plusieurs épitopes et étant présents à une densité comprise entre $10^4$ et $5.10^5$ molécules/µm$^2$ pour chacune des protéines.

L'invention concerne aussi les caractéristiques ci-après, considérées isolément ou selon toutes leurs combinaisons techniquement possibles:

Le couplage des protéines ou peptides antigéniques ou microparticules doit être covalent pour éviter la libération sous forme soluble de l'antigène.

Les microparticules ont un diamètre moyen compris environ entre 0,25 µm et 1,5 µm, et préférentiellement d'environ 1 µm afin de pouvoir être présentées aux lymphocytes T, CD4$^+$ par des cellules phagocytaires mais pas par des lymphocytes B.

Lesdites microparticules sont plus particulièrement caractérisées en ce que la liaison covalente est réalisée par réaction des fonctions NH$_2$ et/ou CO des protéines ou peptides et du matériau constituant la microparticule.

Avantageusement une telle liaison se fera par l'intermédiaire d'un agent pontant, tel que par exemple le glutaraldéhyde ou le carbodiimide. Néanmoins, tout autre agent bifonctionnel permettant une telle liaison peut être utilisé. De tels agents sont connus, voir par exemple Synthetic polypeptides as antigens, M. H. Von Regenmortel, J. P. Briand, S. Muller and S. Plane 1988 (Elsevier). Cette liaison peut également être faite sans agent pontant.

Le matériau constituant la microparticule peut être avantageusement un polymère biocompatible, tel qu'un polymère acrylique, par exemple la polyacroléine ou le polystyrène ou des poly(alpha-acides hydroxyques), des copolymères d'acides lactiques et glycoliques, ou des polymères d'acides lactiques.

On entend par polymère tout homopolymère ou hétéro- ou copolymère.

Il doit permettre un couplage covalent des protéines ou des peptides au matériau et ne doit pas entraîner de réaction de rejet ou de toxicité de la part de l'organisme auquel il pourrait être injecté. Avantageusement, il s'agit, pour l'application en thérapeutique humaine, d'un polymère biodégradable, par exemple un polymère pouvant être dégradé par les cellules possédant des enzymes lysozomiaux, telles que les macrophages.

De tels matériaux biodégradables peuvent être des polymères d'acide lactique et glutamique de l'amidon ou des polymères utilisés pour des usages biomédicaux, et en particulier ceux utilisés dans les sutures.

Une telle microparticule peut porter à sa surface outre des protéines ou des peptides antigéniques, des molécules susceptibles d'activer le système immunitaire, telles que des interleukines, en particulier l'interféron-gamma ou l'interleukine 4.

Ces microparticules peuvent porter une ou plusieurs protéines ou peptides qui peuvent elles-mêmes comprendre chacun un ou plusieurs épitopes. De telles protéines ou peptides peuvent être des glycoprotéines, des peptides synthétiques contenant un épitope ou plusieurs épitopes, ou toute autre molécule non protéique ou contenant une partie protéique pouvant induire une réponse immunitaire.

Les microparticules objets de la présente invention peuvent en outre être encapsulées afin de protéger les antigènes fixés à leurs surfaces d'une dégradation et afin de les amener jusqu'à leur lieu d'action.

Elles peuvent ainsi comprendre un noyau constitué d'une matrice polysaccharidique, à laquelle sont liés les antigènes, d'une première couche lipidique liée de manière covalente au noyau et d'une seconde couche de molécules amphiphiles.

L'invention a pour autre objet des médicaments ou vaccins comprenant les microparticules décrites ci-dessus, ainsi que des compositions pharmaceutiques caractérisées en ce qu'elles les comprennent, en association avec des diluants ou des adjuvants pharmaceutiquement compatibles.

La présente invention est de manière générale relative à l'utilisation de microparticules en matériau synthétique polymère portant en surface une ou plusieurs protéines ou peptides liés de manière covalente, la ou lesdites protéines ou peptides portant chacun un ou plusieurs épitopes T ou B, pour la fabrication d'un médicament ou d'un vaccin pour l'induction d'une réponse immunitaire, selon laquelle les densités de la ou des protéines ou peptides à la surface des microparticules sont ajustées afin d'orienter ladite réponse immunitaire vers une réponse majoritairement humorale ou majoritairement cellulaire.

Sous un autre aspect, l'invention a aussi pour objet un procédé pour la fabrication d'un médicament ou d'un vaccin dont la réponse immunitaire est soit majoritairement humorale soit majoritairement cellulaire, de type Th1 ou Th2, ledit procédé étant caractérisé en ce qu'on fixe de manière covalente sur des microparticules ou billes en matériau synthétique polymère au moins une protéine ou un peptide portant un ou plusieurs épitopes en faisant varier la densité de la protéine ou du peptide fixé à la surface selon le type de réponse désirée.

Afin d'induire une réponse cellulaire et humorale, on utilisera préférentiellement des microparticules présentant une densité pour chacune des protéines ou des peptides portant un épitope d'au minimum $10^5$ et préférentiellement d'environ $5.10^5$ molécules/$\mu m^2$. De telles densités correspondent environ pour une bille d'un diamètre de 1$\mu m$ à des quantités de protéines à la surface de la microparticule respectivement de $10^5$ et $4.10^5$ molécules.

En vue de l'induction d'une réponse majoritairement cellulaire, CD4$^+$, classe II restreinte, on utilisera, préférentiellement, des microparticules présentant une densité pour chacune des protéines ou des peptides portant un ou plusieurs epitopes comprise entre environ $10^4$ et $5.10^4$ molécules de protéines/$\mu m^2$.

Les autres caractéristiques de ces microparticules sont celles mentionnées plus haut concernant les microparticules à haute densité.

Les protéines et antigènes liés de manière covalente aux microparticules dépendent de l'application prévue desdites microparticules.

Elles dépendent également du type de réponse immunitaire que l'on souhaite induire, mais aussi de la maladie ou de l'affection que l'on souhaite traiter ou dont on souhaite prémunir le sujet.

A titre d'exemple, on pourra utiliser les épitopes de la région Pre S2 de l'antigène HBS du virus de l'hépatite virale dont les séquences sont les suivantes:

- épitope T: Pre S:T (120-132)

      **MQWNSTTFHQTLQ**

- épitope B: Pre S:B (132-145)

      **QDPRVRGLYFPAGG**

On peut aussi citer à titre d'exemples les épitopes de la protéine VPI du virus de la poliomyélite dont les séquences sont les suivantes:

- épitope T: C3:T (103-115)

    **KLFAVWKITYKDT**

- épitope B: C3:B (93-103)

    **DNPASTTNKDK**

Un autre exemple de séquence est l'épitope de la boucle V3 de la protéine GP120 du virus HIVI dont la séquence est la suivante:

- épitope T + B: boucle V3

    **INCTRPNNNTRKSIRIQRGPGRAFVTIGKIGNMRQAH CNI**

On utilisera préférentiellement les épitopes B pour induire une réponse humorale à l'aide de microparticules à forte densité et les épitopes T pour induire une réponse majoritairement cellulaire à l'aide de microparticules à faible densité en protéines ou peptides de surface.

De telles microparticules seront injectées aux patients que l'on veut traiter de manière thérapeutique ou prophylactique par les moyens connus de l'homme du métier, par exemple par injection souscutanée, intrapéritonéale, intraveineuse, ou par tout autre moyen permettant d'induire une réponse immunitaire.

On se reportera à ce sujet à Current protocols in immunology, (Edited by J.F. Coligen, A.M. Kruisbeek, D.H. Margulies, E.M. Shevach, W. Strober Wiley-Intersciences Editors) dans lequel sont répertoriées les techniques d'immunisation.

Un des avantages particuliers de la présente invention réside dans le fait qu'elle permet d'induire des réponses immunitaires humorale ou cellulaire sans adjonction d'adjuvants aux billes ou microparticules. Néanmoins, l'adjonction d'adjuvants non toxiques et n'entraînant pas de réaction immunitaire parasite est aussi envisageable dans le cadre d'une utilisation selon la présente invention.

La présente invention est illustrée sans pour autant être limitée par les exemples qui suivent dans lesquels:

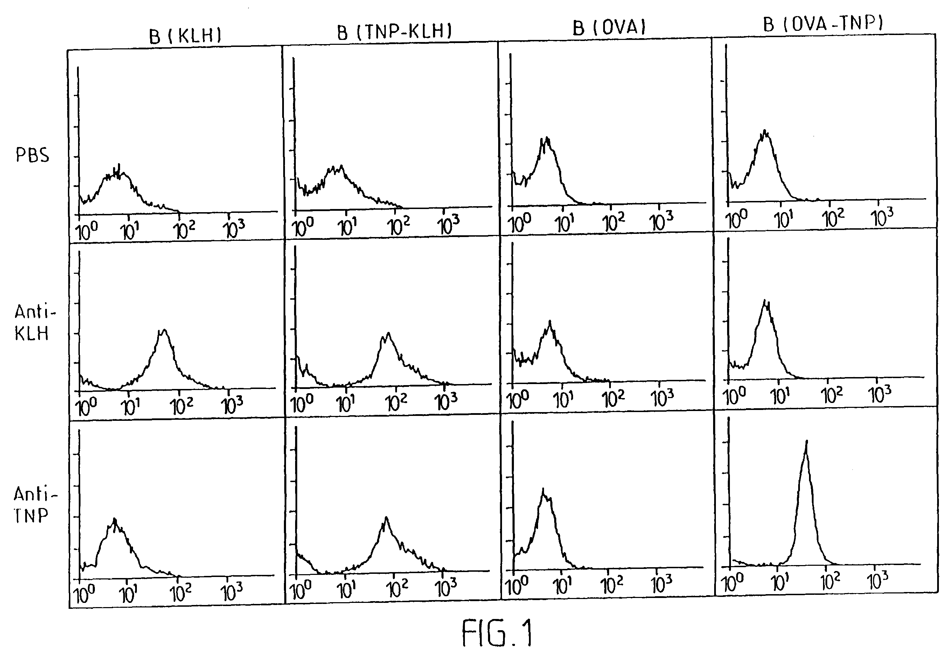

Les diagrammes de la Figure 1 sont des résultats d'analyses par fluorométrie (FACS) de microparticules portant des antigènes KLH ou TNP-KLH. Les ordonnées indiquent l'anticorps utilisé (PBS-témoin, anti-KLH, anti-TNP). Les abscisses indiquent les types de microparticules testées: B (KLH), B (TNP-KLH), B (OVA) et B (OVA-TNP) qui correspondent à des microparticules sur lesquelles sont liés respectivement le KLH, le TNP-KLH, l'ovalbumine et l'ovalbumine-TNP.

Les figures 2A à 2D représentent la capacité de cellules de rate, de macrophages et de cellules spécifiques B du TNP et activées par le LPS à présenter respectivement le KLH, le TNP-KLH, des microparticules portant du KLH et des microparticules portant le TNP-KLH.

La Figure 3 est une courbe indiquant les réponses prolifératives de cellules ganglionnaires de souris immunisées par le lysozyme soluble et stimulées in vitro par le lysozyme soluble (figure 3A) ou par des microparticules portant le lysozyme de diamètres 0,25, 0,75 et 1,5 μm (figure 3B). La prolifération cellulaire est mesurée par l'incorporation de thymidine (CPM) en ordonnées, tandis que la dilution des microparticules est indiquée en abscisses.

La Figure 4 représente la production d'IL2/IL4 par un hybridome spécifique du lysozyme après stimulation par du lysozyme soluble (figure 4A) ou par des microparticules portant du lysozyme (figure 4B). Les concentrations des microparticules sont indiquées en abscisses, tandis que la prolifération est indiquée en ordonnées.

Les Figures 5A et 5B représentent l'activation de l'hybridome T spécifique du lysozyme mesurée par la production d'IL2/IL4 après stimulation par le lysozyme soluble (figure 5A) ou couplé aux microparticules (figure 5B) en présence de splénocytes ou de cellules B(A20) comme cellules présentant l'antigène.

Les Figures 6A et 6B représentent la prolifération in vitro après stimulation par le lysozyme soluble de cellules de ganglions inguinaux de souris immunisées respectivement par du lysozyme soluble en adjuvant complet de Freund (Figure 6A) et par du lysozyme couplé à des microparticules (Figure 6B).

Les figures 7A et 7B représentent la prolifération in vitro après stimulation par le lysozyme de cellules de ganglions inguinaux de souris immunisées par diverses concentrations de lysozyme soluble (7A) ou couplé à des microparticules (7B).

La figure 8 représente la prolifération de cellules ganglionnaires de souris immunisées par le lysozyme en adjuvant complet de Freund ou en PBS ou an présence de microparticules couplées à de l'hémocyanine de patelle, ou KLH (Keyhole Limpet Hemocyanin).

La Figure 9 représente la réponse proliférative de cellules ganglionnaires de souris immunisées par de l'hémoglobine soluble an adjuvant (Hb + CFA) ou couplée à des billes (B-Hb).

La Figure 10 représente la réponse proliférative de cellules de souris stimulées par de l'ovalbumine soluble (OVA + CFA) ou de l'ovalbumine couplée à des billes (B-OVA).

La réponse proliférative, respectivement à l'hémoglobine et à l'ovalbumine , a été mesurée en ordonnées par la radioactivité incorporée (CPM), en fonction de la quantité d'hémoglobine ou d'ovalbumine (an abscisses) utilisée pour restimuler les cellules.

La Figure 11 illustre la prolifération de cellules de souris immunisées par le peptide C3 sous forme soluble (C3:T + CFA) ou sous forme microparticulaire (B-C3: T). Les cellules ont été restimulées en présence de quantité de C3 soluble (en abscisses) et la prolifération a été mesurée (en ordonnées).

La Figure 12 représente la prolifération des cellules de souris immunisées par le peptide pré-S:TB soluble (Pré-S:TB + alum) ou sous forme microparticulaire (B-pré-S:TB) ou le peptide pré-S:B sous forme particulaire (B-Pré S:B) et restimulées par le peptide Pré-S.

Les Figures 13A et 13B représentent respectivement les taux d'anticorps anti-lysozyme (Figure 13A) et d'anticorps anti-KLH (Figure 13B) de souris immunisées avec du lysozyme et de l'adjuvant alun, des microparticules portant du lysozyme ou des microparticules portant du LH.

Les Figures 14 et 15 illustrent la réponse anticorps de souris immunisées par l'hémoglobine (Figure 14) ou l'ovalbumine (Figure 15) sous forme soluble ou particulaire. Le Log du titre en anticorps est représenté en ordonnées tandis que le temps est représenté en abscisses.

La Figure 16 est relative à la réponse anticorps de souris immunisées par le peptide pré-S : TB soluble ou les peptides pré-S: TB ou pré-S: B sous forme particulaire. L'ordonnée et l'abscisse de cette courbe ont la même signification que pour les Figures 14 et 15.

La Figure 17A représente la prolifération de cellules de souris immunisées par injection de lysozyme en présence d'adjuvant de Freund, après stimulation in vitro par des microparticules portant du lysozyme à différentes densités.

La Figure 17B représente la prolifération in vitro de cellules de souris immunisées in vivo par du lysozyme ou des billes portant du lysozyme après stimulation par différentes concentrations de lysozyme.

La Figure 18 est un diagramme illustrant la production d'anticorps anti-lysozyme de cellules de souris immunisés par injection de lysozyme et d'adjuvant de Freund ou de microparticules portant du lysozyme.

EXEMPLE 1:

Préparation de billes couplées à KLH ou à l'ovalbumine.

1. Matériel et méthodes et présentation par des cellules B ou par des macrophages.

Les souris sont des femelles BALB/c et DBA/2 âgées de 6 à 8 semaines.

Les antigènes sont le KLH et l'ovalbumine (OVA) commercialisés par Sigma Chemical (St-Louis, USA). L'hémocyanine trinitrophénylée (TNP4-KLH) a été préparée telle que décrit précédemment (Shutze et al., J. Immunol. (1989) 142:2635).

1.1 Couplage covalent des antigènes ou microparticules de poly(acroléine).

Des microparticules de poly(acroléine) d'un diamètre compris entre O,25 et l,5 μm, commercialisées par Polysciences Inc. (Washington PA), sont couplées à l'ovalbumine ou au KLH comme décrit précédemment (Rembaum et al. Immunol. (1982) 52:341; Ziegler et al. Eur. J. Immunol. (1987) 17:1287).

1 ml de ces microparticules est lavé deux fois dans du PBS et resuspendu dans l ml de KLH ou d'ovalbumine (5 mg/ml dans du PBS). Après trois heures d'incubation à température ambiante, les microparticules sont lavés deux fois dans du PBS et resuspendus dans 2 ml de PBS contenant 1% de sérum albumine bovine (BSA) et des antibiotiques. Les microparticules ainsi obtenues sont stockées à 4°C jusqu'à utilisation.

Les microparticules portant les antigènes TNP-OVA ou TNP-KLH sont préparées par incubation de microparticules portant l'OVA ou le KLH avec du TNBS (Trinitrobenzène sulfonate).

2 ml des microparticules qui ont été couplées au KLH ou à l'ovalbumine sont lavés deux fois dans du PBS et resuspendus dans 2 ml de tampon cacodylate contenant 10 mg/ml de TNBS. Les microparticules sont incubées 3O minutes dans l'obscurité à la température ambiante et lavées trois fois dans du PBS. Elles sont resuspendues dans 2 ml de PBS contenant 1% de BSA et des antibiotiques et stockées à 4°C.

1.2 Analyse par cytofluorométrie de flux.

5O μl de microparticules sont lavés deux fois dans du PBS contenant 1% de BSA et incubés durant 4O minutes à 4°C avec du sérum de souris anti-KLH ou anti-TNP. Après deux lavages, les microparticules sont incubées avec des anticorps de chèvre couplés au FITC (fluoroisothiocyanate) dirigés contre des immunoglobulines de souris (Biosys, Compiègne, France) durant 4O minutes à 4°C.

Après quatre lavages, les microparticules sont resuspendues dans l ml de PBS contenant l% de BSA.

L'intensité de fluorescence est mesurée on utilisant le cytomètre de flux FACSCAN (Becton Dickinson, Mountain View.CA).

1.3 Milieu de culture.

Les lymphocytes sont cultivés dans du RPMI 1640 (Seromed, Munich, FRG) complémentés avec de la L-glutamine 2mM, IO% de FCS (sérum de veau foetal) inactivé par la chaleur, du 2-ME 5OμM et des antibiotiques.

### 1.4 Etablissement de la lignée cellulaire Th spécifique du KLH.

Cette lignée cellulaire est établie et maintenue selon le protocole décrit par Taylor et al. (ed. IRL Press. New York) et Galelli et al. (J. Immunol. (1990) 145:2397).

Des cellules de ganglions inguinaux (4 $IO^6$/ml) de souris DBA/2 ayant subi 8 jours avant le prélèvement des cellules, une injection de 100 μg de KLH en émulsion dans de l'adjuvant complet de Freund à la base de la queue ont été cultivées durant 4 jours dans du milieu de culture en présence de KLH (lOO μg/ml).

Les cultures sont incubées dans une atmosphère humide à 7,5% de $CO_2$ à 37°C.

Une lignée cellulaire a été établie à partir de cette culture initiale par des passages en série de cellules T purifiées sur Ficoll (2.$IO^5$/ml) en présence de cellules de rate de souris DBA/2 irradiées (3OOO rad) durant 6 à 8 jours (période de repos) ou avec des cellules de rate irradiées plus du KLH (lOO μg/ml) durant 4 jours (période de stimulation).

Les cellules T utilisées dans les expériences sont récoltées 8 à IO jours après leur dernière mise en présence du KLH.

### 1.5 Estimation de la prolifération des cellules Th.

Des cultures en triplicats contenant 5.$IO^4$ cellules Th purifiées sur Ficoll, et 5.$IO^4$ cellules B mémoire spécifiques du TNP purifiées et irradiées (9OO rad), ou 5.$IO^5$ cellules de rate totales irradiées (3OOO rad), ou IO.$^5$ cellules de rate adhérentes irradiées (3OOO rad), ou $IO^5$ cellules B de lymphome A2O positives pour le MHC de classe II irradiées (33OO rad) (Kim et al. J. Immunol. (1979) 122:549), ou $IO^5$ cellules B vierges spécifiques du TNP et activées par le LPS comme source de cellules présentant les antigènes, et différentes concentrations d'antigène ont été incubées dans des plaques de microculture à fond plat (Corning, Cambridge, MA) sous un volume total de O,2 ml/puits de milieu complet. La prolifération cellulaire T a été estimée par incorporation de la thymidine tritiée durant les huit dernières heures d'une culture de 3 jours.

Les résultats sont exprimés comme la moyenne géométrique de trois cultures, une fois éliminé le bruit de fond. L'écart type est inférieur à 15 % de la moyenne.

### 1.6 Cellules B spécifiques du TNP.

Les cellules B spécifiques du TNP de souris normales sont purifiées par liaison et élution sur une gélatine-TNP8 et selon la technique décrite par Haas et Layton J. E., J. Exp. Med. (1975) 141:1004.

Ce protocole a été modifié afin d'obtenir des populations enrichies en cellules B mémoires spécifiques du TNP à partir de la rate de souris précocement immunisées, comme précédemment décrit (Galelli et al. J. Immunol. (1990) 145:2397). Les cellules B mémoire spécifiques du TNP ont été sélectionnées sur de la gélatine portant un haptène (gélatine-TNP2), en testant l'affinité des récepteurs pour le TNP par comparaison aux cellules B vierges, et la capacité à sécréter de larges quantités d'immunoglobulines G anti-TNP en présence de faibles concentrations d' antigènes.

$IO^8$ cellules de rate ne contenant ni érythrocytes ni cellules mortes ont été suspendues dans 3 ml de HEPES (5O mM) tamponnées par du DMEM (Seromed, Munich, Allemagne) et incubées dans des boîtes de Pétri en plastique recouvertes de gélatine-TNP2. Les boites sont agitées de manière douce durant 15 minutes à 4°C, puis lavées IO fois par du DMEM à la température de la glace. Les cellules adhérentes sont éluées en ajoutant 5 ml de DMEM réchauffé à 37°C et la gélatine-TNP liée est éliminée par digestion par la collagénase (Collagenase CLSIII de Worthington Biochemicals Freehold, New Jersey, lOO U/ml) durant 15 minutes à 37°C.

Ce protocole conduit à l'obtention finale, exprimée comme un pourcentage par rapport au nombre de cellules de rate d'origine, de O,3 à O,6 % de cellules liant le TNP à partir de rate de souris immunisées. Les cellules sont cultivées toute la nuit avant l'addition d'autres cellules et réactifs afin de permettre la réexpression d'immunoglobulines de surface modifiées par le traitement par la collagénase. La présence de récepteurs libres du TNP sur ces cellules est évaluée par leur capacité à lier des érythrocytes portant à leur surface du TNP.

55 à 76 % des cellules obtenues à partir de souris immunisées forment des rosettes avec des SRBC modifiées par le TNP. Ces cellules ne prolifèrent pas en réponse à la concanavaline A mais sont enrichies 2O fois, pour les cellules qui secrètent les immunoglobulines G anti-TNP après stimulation par TNP-LH, par comparaison aux cellules de rate non fractionnées.

### 1.7 Cellules B vierges spécifiques du TNP et activées par le LPS

Des cellules B vierges spécifiques du TNP de souris et non immunisées ont été purifiées par liaison puis élution sur de la gélatine-TNP8 comme décrit précédemment. Ces cellules ont été cultivées à une densité de 2.$IO^6$ par ml dans un milieu contenant 5O μg/ml de LPS (Salmonella enteriditis, Difco Laboratories, Détroit, MI) durant 3 jours. Les lymphoblastes non adhérents ont été purifiés en utilisant du Ficoll-Hypaque (Pharmacia, Piscataway, NJ), puis lavés et utilisés comme cellules accessoires.

### 1.8 Macrophages.

Les macrophages ont été obtenus à partir de cellules de rate non immunisées par adhésion durant 4 heures à 37° C suivie d'un lavage des cellules afin d'éliminer les cellules non adhérentes tel que décrit précédemment (Kakiochi et al. J. Immunol. (1983) 131:109).

2. Résultats.

2.1 Vérification du couplage de l'antigène aux microparticules.

Le KLH a été couplé de manière covalente à des microparticules de polyacroléine d'un diamètre de O,25 à l,5 μm. Le couplage du KLH aux microparticules a été contrôlé par analyse en cytofluorométrie de flux en utilisant un sérum de souris anti-KLH.

Les résultats obtenus avec des microparticules de l,5 μm sont indiqués sur la Figure I.

Les microparticules de l,5 μm ont été couplées à l'ovalbumine (B OVA) ou à la KLH (B-KLH). Les microparticules TNP-OVA ou TNP-KLH (respectivement désignées B(TNP-OVA) et B (TNP-KLH)) ont été préparées par incubation de microparticules portant l'OVA ou le KLH avec du TNBS. L'analyse par cytofluorométrie a été effectuée mur des microparticules incubées en présence de PBS ou en présence de sérum de souris anti-KLH ou anti-TNP. Après lavage, les microparticules ont été incubées avec des anticorps de chèvre liés au FITC dirigés à l'encontre des immunoglobulines de souris et ont été analysées par cytométrie de flux.

Des résultats similaires ont été obtenus avec des microparticules de O,25 et O,75 μm.

Les microparticules témoins couplées avec de l'ovalbumine n'ont pas été reconnues par le sérum anti-KLH.

2.2. Comparaison de la capacité de diverses populations de splénocytes à présenter des antigènes solubles ou particulaires.

La capacité de splénocytes non fractionnés, de macrophages et de cellules B vierges spécifiques pour le TNP a été comparée quant à leur présentation de KLH et de TNP-KLH soluble ou particulaire à des cellules T spécifiques du KLH.

Dans ces expériences, des populations de splénocytes ont été préparées à partir de souris non immunisées. Après purification, les cellules B spécifiques du TNP ont été activées durant trois jours par du LPS; on sait en effet que les lymphoblastes induits par le LPS sont des cellules très efficaces quant à la présentation d'un antigène (Kakiochi et al. J. immunol. (1983) 131:109).

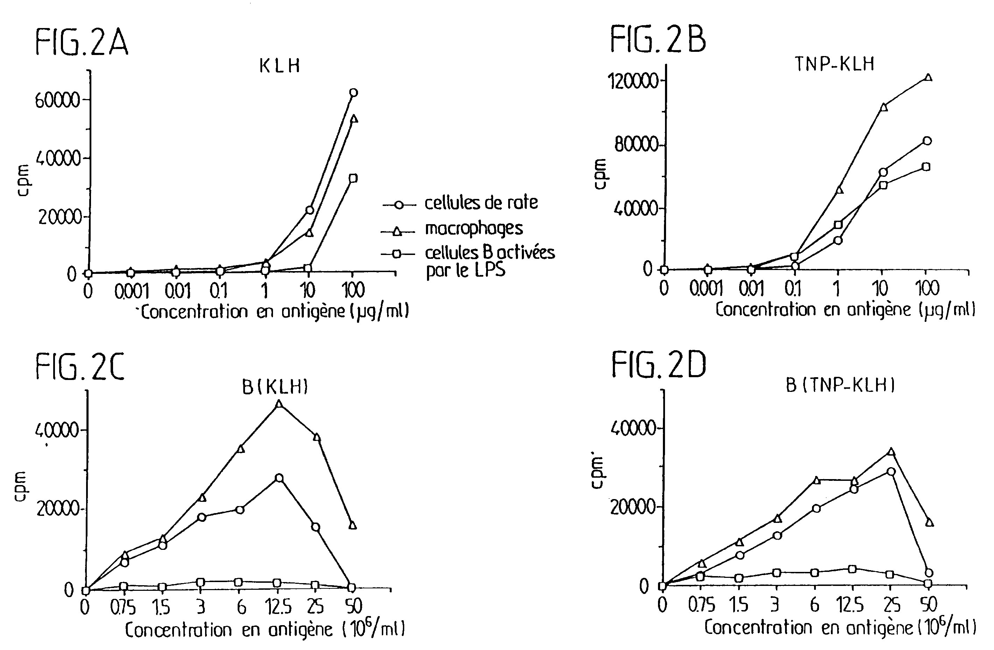

Les résultats sont illustrés sur la Figure 2 pour laquelle 5.$IO^5$ splénocytes irradiés, $IO^5$ cellules adhérentes ou $IO^5$ cellules B vierges spécifiques du TNP activées par le LPS ont été cultivées avec 5.$1O^4$ cellules Th spécifiques du KLH en présence de quantités variées de KLH soluble (A), de TNP-KLH soluble (B) ou fixés sur des microparticules (B KLH) (C) ou de B (TNP-KLH) (D)). La prolifération cellulaire Th a été estimée au jour 3.

Comme le montre la Figure 2 (2A et 2B), les macrophages et les cellules B activées par le LPS stimulent de manière efficace les cellules T quand ils sont incubées avec du KLH ou du TNP-LH solubles.

Contrairement à ces résultats, seuls les macrophages, et non les cellules B spécifiques du TNP et activées par le LPS sont capables de stimuler les cellules T spécifiques du KLH (Figures 2 C et D) quand des microparticules portant du KLH ou du TNP-KLH sont utilisées.

Ces résultats montrent que les macrophages sont responsables de l'activité de présentation de l'antigène des cellules spléniques quand des antigènes particulaires sont utilisés.

Ainsi l'incapacité de cellules B spécifiques du TNP à présenter l'antigène particulaire a été illustrée.

EXEMPLE 2.

Induction d'une réponse proliférative T, CD4$^+$ spécifiques du lysozyme in vivo et in vitro par des microparticules couplées au lysozyme.

1. MATERIELS ET METHODES.

1.1 Antigènes

Le lysozyme (LYSO) et l'hémocyanine de Limulus (LH) proviennent des Laboratoires Sigma.

1.2 Couplage de l'antigène aux microparticules

L'antigène soluble est rendu particulaire par couplage à des microparticules (Polysciences) de O.2 à 1μm de diamètre. Deux méthodes de couplage sont utilisées:

1.2 a) Couplage covalent directement sans agent activant.

Les billes ou microparticules de polyacroléine portent des groupements aldéhyde capables de réagir spontanément avec les fonctions amines des protéines.

l ml de billes est lavé 4 fois dans du PBS, et repris dans l ml d'antigène à 5 mg/ml. Après 3 heures d'incubation à température ambiante, les billes sont lavées 3 fois dans du PBS et incubées 3O minutes dans l ml de PBS-Albumine humaine l% afin de saturer les groupements réactifs libres des billes. Puis après lavage, les particules sont reprises dans 2 ml de PBS-albumine humaine l%-antibiotique l% puis conservées à +4°C.

b) Couplage covalent par le glutaraldéhyde.

L'antigène est couplé aux billes de polystyrène par le glutaraldéhyde, capable de former une base de Schiff avec les groupements amines des protéines.

O,5 ml de billes est lavé 3 fois dans du PBS et repris dans O,5 ml de glutaraldéhyde 8%. Après 6 heures d'incubation à température ambiante, les billes sont lavées 2 fois et reprises dans l ml d'antigène à 4OOµg/ml. Après incubation pendant la nuit à température ambiante, les billes sont lavées et incubées avec l ml d'éthanolamine O,2 M pendant 3O minutes afin de bloquer les fonctions aldéhyde libres du glutaraldéhyde.

Après un dernier lavage, les particules sont reprises dans l ml de PBS- albumine humaine l%-antibiotique l% puis conservées à +4°C.

Cette méthode de couplage permet de déterminer la quantité de protéines couplées sur les microparticules par spectrophotométrie. Les absorbances de la solution de protéine à 4OO µg/ml et du surnageant obtenu après l'incubation des billes avec cette solution de protéine sont mesurées à 28O nm. Connaissant le nombre de billes utilisées pour le couplage, on considère que la différence entre la quantité de protéine avant couplage et la quantité résiduelle après couplage, permet d'estimer la quantité de lysozyme couplée par particule.

1.3 Protocole d'immunisation.

On a utilisé des femelles BALB/c, d'haplotype H-2$^d$, âgées de 6 à 8 semaines (élevage de l'Institut Pasteur).

- immunisation par voie intra-péritonéale: on injecte lOO µg de lysozyme avec l mg d'alum, ou, différentes quantités d'antigène couplé aux billes sans adjuvant,
- immunisation par voie sous-cutanée: à la base de la queue, on injecte lOO µg de lysozyme en émulsion dans l'adjuvant complet de Freund, ou, différentes quantités d'antigène couplé aux billes.

Le sérum de chaque souris est prélevé 7 ou 14 jours après chaque injection. La teneur en anticorps des sérums est mesurée par test ELISA.

La réponse proliférative cellulaire est mesurée sur les ganglions inguinaux et/ou sur la rate, prélevés 7 et/ou l4 jours après chaque injection.

1.4 Détection des anticorps par ELISA.

L'antigène (lysozyme) est incubé à la concentration de 5µg/ml on tampon carbonate 5O mM-pH=9.6, dans des microplaques (Nunc) pendant une nuit à 4°C. Après lavage avec un tampon PBS-Tween 2O à O,O1%, les différentes dilutions des sérums à tester, réalisées en tampon BSA l%, sont incubées pendant l heure à 37°C. Après lavage, on dépose lOO µl par puits d'un conjugué anti-Ig de souris (anti-Ig totales fournis par Diagnostics Pasteur et anti-Ig spécifiques par Sigma), marqué à la peroxydase, préparé chez la chèvre; qui est incubé pendant l heure à 37°C. Après lavage, on ajoute la solution de substrat préparée extemporanément: Orthophénylènediamine à O,5 mg/ml (Sigma) on tampon acide citrique O.1 M-phosphate disodique O.2M-pH=5 auquel on ajoute H$_2$O$_2$ au l/25OO.

Une coloration jaune révèle la présence d'anticorps spécifiques; la réaction enzymatique est arrêtée 8 minutes plus tard, par 5O µl de H$_2$SO$_4$ (l1,5%).

L'absorbance de chaque puits est mesurée à 492 nm, par un lecteur de densité optique (Dynatech). Le contrôle négatif est réalisé avec du sérum au l:lOO de souris BALB/c non immunisées. Les résultats sont exprimés: soit on DOxlOOO à partir de l'absorbance mesurée, corrigée de l'absorbance on absence de sérum; soit par le titre on anticorps calculé à partir de régression linéaire basée sur l'absorbance obtenue avec le sérum de souris BALB/c non immunisées.

Lorsque l'antigène est sous forme particulaire, le test ELISA est réalisé on tubes. Les dilutions des sérums à tester sont incubées directement avec l'antigène couplé aux billes (8.lO$^8$ particules/ml). Les lavages se font par centrifugation dans le tampon PBS-Tween 2O (O.O1%). Lorsque la réaction enzymatique est terminée, 2OO µl de chaque tube sont transférés en microplaque puis l'absorbance est mesurée.

1.5 Inhibition de la fixation des anticorps anti-lysozyme par test ELISA.

Le test ELISA mesure la fixation des anticorps spécifiques présents dans le sérum de souris BALB/c immunisées par le lysozyme. Cette fixation est diminuée si le sérum est préincubé (avant le test ELISA) avec l'antigène: lysozyme soluble ou couplé aux billes, qui se comporte alors, comme un inhibiteur.

Le sérum anti-lysozyme est préincubé avec le lysozyme soluble ou couplé aux billes, pendant l heure à 37°C puis la nuit à 4°C; la réaction se faisant en tubes. La fixation des anticorps non-liés à l'inhibiteur est évaluée par test ELISA (triplicats) en microplaques, dont les puits ont été recouverts par du lysozyme à 5 µg/ml. L'absorbance de chaque puits est mesurée à 492 nm, et corrigée de l'absorbance en absence de sérum. Le contrôle négatif est réalisé avec du sérum au l:lOO de souris BALB/c non immunisées. L'absorbance sans inhibiteur lors de la préincubation de sérum, correspond à la fixation maximale d'anticorps anti-lysozyme.

Les résultats sont exprimés en pourcentage d'inhibition de fixation des anticorps et calculé selon le rapport

$$\frac{\text{DO sans inhibiteur-DO avec inhibiteur}}{\text{DO sans inhibiteur}}$$

La détermination graphique de la concentration de lysozyme soluble ainsi que du nombre de billes couplées au lysozyme, nécessaire pour 50% d'inhibition, permet d'estimer la quantité de lysozyme fixée par particule.

1.6 Stimulation d'un hybridome T spécifique du lysozyme

Un hybridome T a été produit par immunisation de souris BALB/c avec du lysozyme. Il reconnaît spécifiquement le peptide 108-116 du lysozyme, en association avec les molécules du Complexe Majeur d'Histocompatibilité de classe II I-E[d].

$10^5$ cellules d'hybridome T sont stimulées par des concentrations croissantes d'antigène: lysozyme ou billes couplées, en présence de différentes cellules présentatrices de l'antigène: $5.10^5$ splénocytes irradiés (3000 rad) de souris BALB/c ou $10^5$ cellules de lymphome B A20, restreintes par les molécules de CMH de classe II. Les cellules sont mises en culture (triplicats) dans un milieu complet RPMI 1640 (SEROMED) additionné de 10% de sérum de veau foetal décomplémenté, 50 $\mu$M de $\beta$-mercaptoéthanol, 2mM de glutamine, 100 UI/ml de pénicilline et 100$\mu$g/Ml de streptomycine, en microplaque à fond plat (Corning 25860). Le témoin positif est réalisé par stimulation de l'hybridome par le mitogène de lymphocytes T: concanavaline A à 5 $\mu$g/ml.

Le surnageant est prélevé après 24h de culture à 37°C (7.5%$CO_2$), puis congelé à - 20°C pendant 16h minimum. La stimulation de l'hybridome est mesurée par la teneur en IL2 du surnageant dans un test de prolifération de cellules CTL-L. Les écarts types ne sont pas mentionnés, car l'erreur est inférieure à 10% de la moyenne des triplicats.

1.7 Dosage de l'IL2 et de l'IL4

La lignée CTL-L est dépendante de l'Interleukine 2 et de l'Interleukine 4; elle est maintenue en culture en milieu complet enrichi de 20% de surnageant de splénocytes de rat, incubés 36h avec 2,5 $\mu$g/ml de concanavaline A.

Après décongélation, les surnageants de cultures (testés au 1/2) sont incubés en présence de $2,25.10^4$ cellules CTL-L, préalablement lavées trois fois dans le milieu RPMI1640, pendant 3 jours à 37°C (7,5% $CO_2$).

La prolifération cellulaire est mesurée par addition de thymidine tritiée d'activité spécifique l Ci/mmole, à raison de 2 $\mu$Ci/ml de culture, pendant les 16 dernières heures de culture.

L'ADN des cellules est récupéré après lyse des cellules et filtration à l'aide d'un "Skatron". L'incorporation de radioactivité est comptée par scintillation à l'aide d'un compteur-bêta.

Les résultats sont exprimés en cpm à partir de la moyenne des triplicats, corrigée de la radioactivité incorporée en l'absence d'antigène.

1.8 Test de prolifération

La rate et/ou les ganglions inguinaux sont prélevés stérilement 7 ou 14 jours après l'immunisation des souris (voir protocole d'immunisation). $8.10^5$ cellules sont incubés on présence de différentes concentrations d'antigène, soluble ou couplé aux billes. Les cellules sont mises en culture (triplicats) dans du milieu RPMI 1640 (SEROMED) additionné de 1.5% de sérum de veau foetal décomplémenté, 0,5% de sérum normal de souris, 50 $\mu$M de $\beta$2-mercaptoéthanol, 2mM de glutamine, 100 UI/ml de pénicilline et 100 $\mu$g/ml de streptomycine; en microplaques (Corning 25860) pendant 4 jours à 37°C (7,5% $CO_2$).

La prolifération des cellules est mesurée par incorporation de thymidine tritiée, d'activité spécifique 25 Ci/mmole, à raison de 2 $\mu$Ci/ml de culture, pendant les 16 dernières heures. L'ADN des cellules est récupéré après lyse des cellules et filtration à l'aide d'un Skatron, l'incorporation de radioactivité est comptée par scintillation à l'aide d'un compteur-bêta.

Les résultats sont exprimés en cpm à partir de la moyenne des triplicats, corrigée de l'incorporation en absence d'antigène.

2 - RESULTATS.

2.1. Stimulation par le lysozyme couplé aux microparticules de cellules ganglionnaires de souris immunisées par le lysozyme.

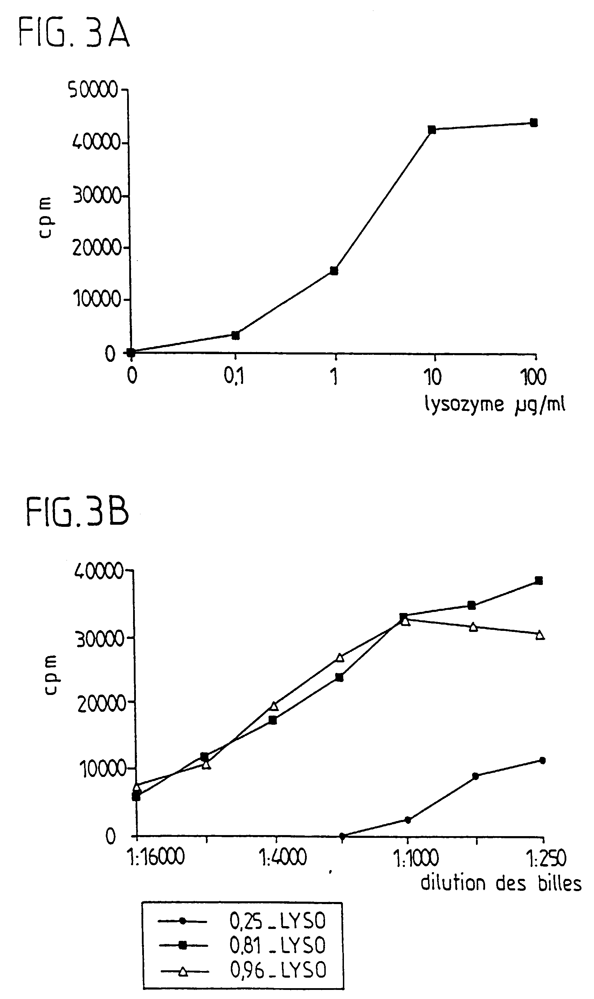

Dans les essais illustrés aux Figures 3A et 3B, des souris BALB/c ont été immunisées par injection sous-cutanée à la base de la queue avec du lysozyme soluble complémenté avec de l'adjuvant de Freund (CFA).

Après 14 jours, les ganglions inguinaux ont été prélevés, et la réponse proliférative de ces cellules a été testée in vitro contre différentes concentrations de lysozyme ou contre différentes concentrations de microparticules couplées au lysozyme. Les résultats sont exprimés en cpm corrigés de la valeur obtenue sans antigène.

Le lysozyme soluble induit une prolifération importante des cellules de souris immunisées par cet antigène en adjuvant de Freund (3A). La stimulation in vitro de ces mêmes cellules par les microparticules-lysozyme révèle que celles-ci sont capables d'induire une très forte prolifération cellulaire (figure 3B). Les microparticules de plus grand diamètre, O,81 et 0,96 $\mu$m (couplage spontané), sont très efficaces.

2.2. <u>Stimulation par le lysozyme couplé aux billes de l'hybridome T.</u>

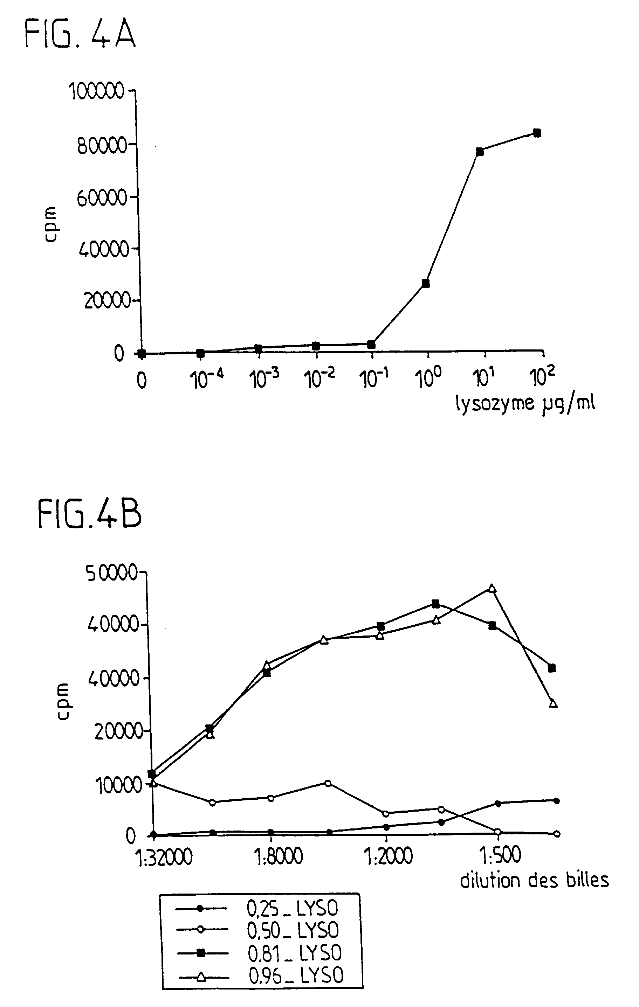

Les figures 4A et 4B correspondent aux résultats de stimulation de l'hybridome T, spécifique du lysozyme par le lysozyme soluble (4A) ou couplé aux microparticules (4B). Le degré de stimulation de l'hybridome a été mesuré par le taux d'IL-2/IL-4 produites.

En présence de splénocytes irradiés, l'hybridome T est stimulé fortement par le lysozyme soluble (figure 4A). En présence de ces cellules, les microparticules-lysozyme de grande taille (O,81 et 0,96 μm) entraînent également une production d'IL-2/IL-4 importante (figure 4B), contrairement aux microparticules de O,5 et O,25 μm qui ne sont pas capables de stimuler l'hybridome T spécifique.

2.3. <u>Incapacité des cellules A20 de lymphome B à présenter le lysozyme couplé aux billes à l'hybridome T, spécifique du lysozyme.</u>

On sait que des tumeurs cellulaires B portant des récepteurs Ia peuvent être utilisés comme cellules présentant des antigènes pour des antigènes qui n'ont pas de réactivité avec le récepteur Ig mais qui sont fixés par les tumeurs de cellules B par des mécanismes non spécifiques (Walker et al. J. Immunol. (1982) 128:2164; Glimcher et al. J. Exp. Med. (1982) 155:445; Mac Kean et al. J. Exp. Med. (1981) 154:1419; Mac Kean et al. J. Exp. Med. (1981) 154:1419).

On a donc testé la capacité d'une de ces tumeurs cellulaires B, la lignée A2O, à présenter le lysozyme sous forme soluble ou particulaire.

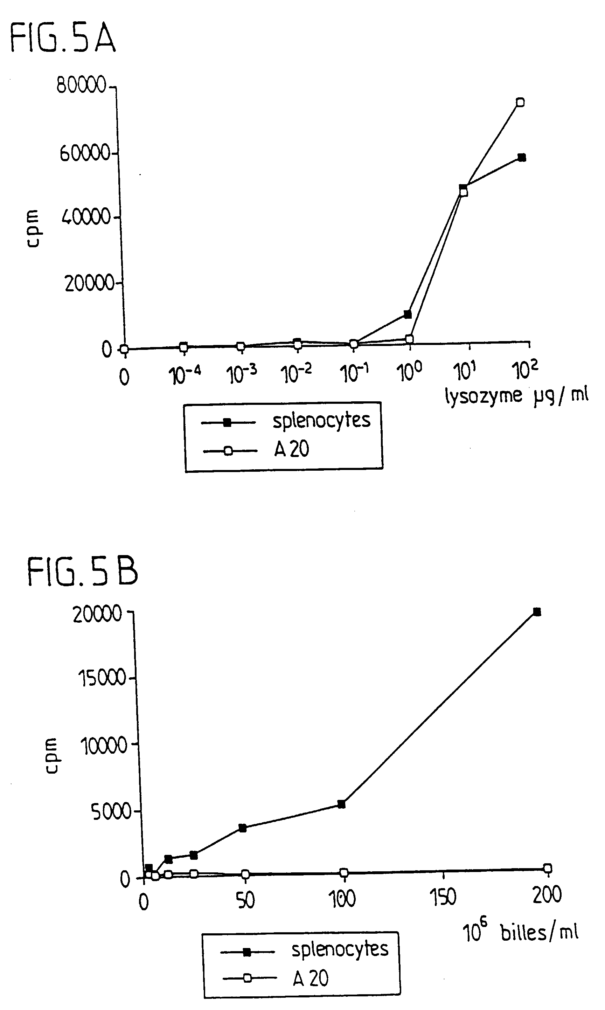

La présentation du lysozyme soluble ou particulaire a été comparée en utilisant deux sources de CPA: soit une source hétérogène, les splénocytes totaux irradiés, soit des cellules B provenant du lymphome A2O. Lorsque l'antigène est sous forme soluble (figure 5A), il peut stimuler l'hybridome T aussi bien en présence de splénocytes que des cellules B A2O. Au contraire, le lysozyme particulaire est présenté uniquement par les splénocytes et non par les cellules B A2O (figure 5 B).

Ces résultats confirment que les splénocytes peuvent présenter aux cellules T un antigène, qu'il soit soluble ou sous forme particulaire. Par contre, les lymphocytes B sont incapables de présenter un antigène rendu particulaire par couplage à des billes d'une taille de l'ordre du micron.

2.4 <u>Induction de réponses T prolifératives par injection à des souris de lysozyme couplé aux microparticules.</u>

L'immunogénicité in vivo de l'antigène couplé aux microparticules a été analysée en immunisant des souris BALB/c avec du lysozyme en adjuvant complet de Freund ou avec cet antigène couplé à des billes de polyacroléine. Après 14 jours, les cellules des ganglions drainants de ces animaux ont été stimulées in vitro par différentes concentrations de lysozyme soluble.

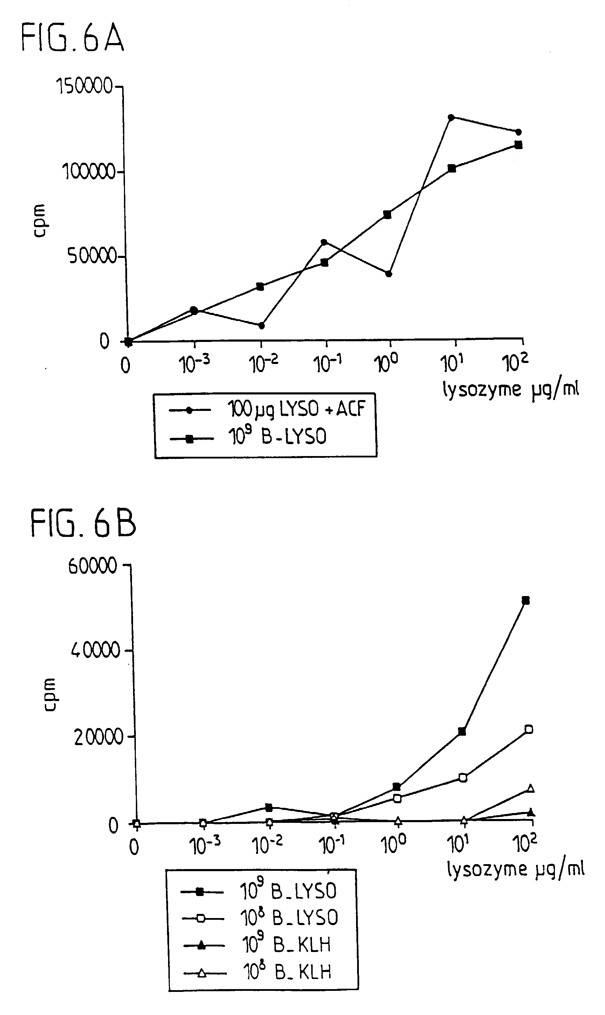

En présence de lysozyme soluble, les cellules ganglionnaires prolifèrent fortement, qu'elles proviennent de souris immunisées avec le lysozyme soluble ou avec des microparticules-lysozyme (figure 6A). Ceci démontre que dans les deux cas, des cellules T spécifiques du lysozyme ont été sensibilisées in vivo. Après injection à des souris de microparticules-LH représentant le contrôle de spécificité, les cellules ganglionnaires de ces animaux sont incapables de proliférer en réponse à une stimulation par le lysozyme soluble in vitro (figure 6B). La réponse cellulaire in vitro est donc spécifique de l'antigène protéique couplé aux microparticules, utilisé lors de l'immunisation des souris.

La réponse proliférative des cellules sensibilisées par $IO^9$ microparticules-lysozyme (correspondant à 1 μg de lysozyme) en absence d'adjuvant, est aussi élevée que celle des cellules d'animaux immunisés par 100 μg de lysozyme soluble en adjuvant de Freund (CFA) (figure 6A). Pour vérifier et préciser ce résultat, les réponses prolifératives des cellules ganglionnaires d'animaux ayant reçu différentes doses de lysozyme en CFA ou différentes concentrations de microparticules couplées ont été comparées, après stimulation in vitro par le lysozyme soluble.

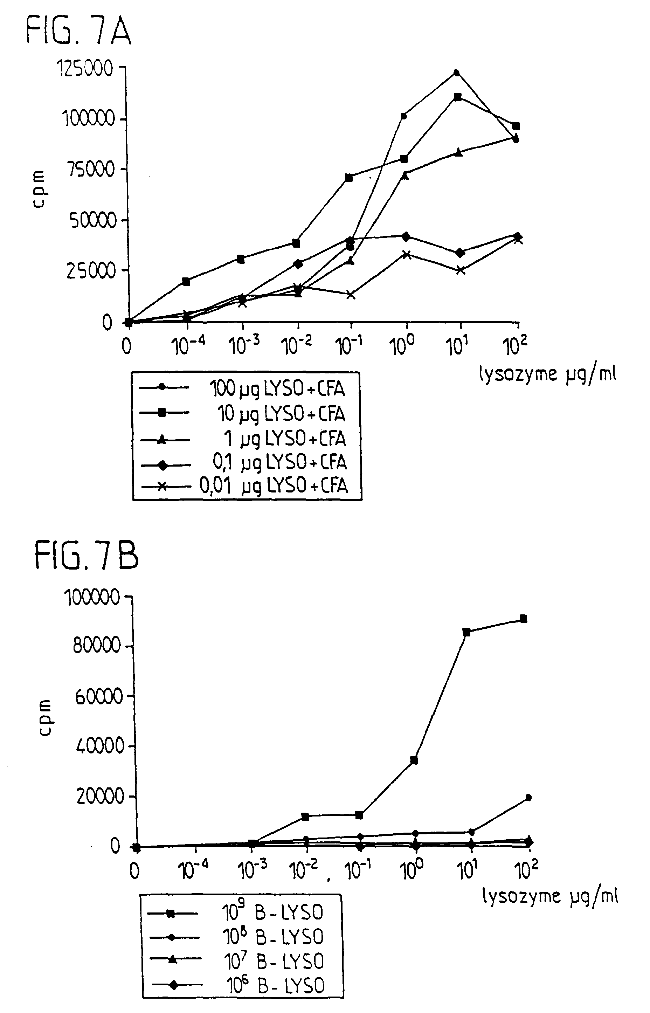

Dans le cas des Figures 7A et 7B, des souris ont été immunisées par injection sous-cutanée à la base de la queue avec du lysozyme soluble et de l'adjuvant complet de Freund (CFA) (Figure 7A) ou des billes couplées à l'antigène sans aucun adjuvant (Figure 7B).

Après 14 jours, les ganglions inguinaux ont été prélevés, et la réponse proliférative de ces cellules a été testées in vitro contre différentes concentrations de lysozyme. Les résultats sont exprimés en cpm corrigés de la valeur obtenue sans antigène.

Sur la Figure 7B, il est à noter que les dénominations $10^9$, $10^8$, $10^7$ et $10^6$ B-LYSO correspondent respectivement à des poids de 1; 0,1; 0,01 et 0,001 μg; en lysozyme.

Ces résultats démontrent que les cellules ganglionnaires des animaux immunisés avec des microparticules portant du lysozyme prolifèrent in vitro après mise en contact avec le lysozyme, indiquant ainsi une sensibilisation des cellules T spécifiques de cet antigène.

La comparaison des effets doses (Figure 7) indique que l μg de lysozyme couplé aux billes donne une réponse quasi-équivalente à celle de l μg d'antigène injecté en CFA.

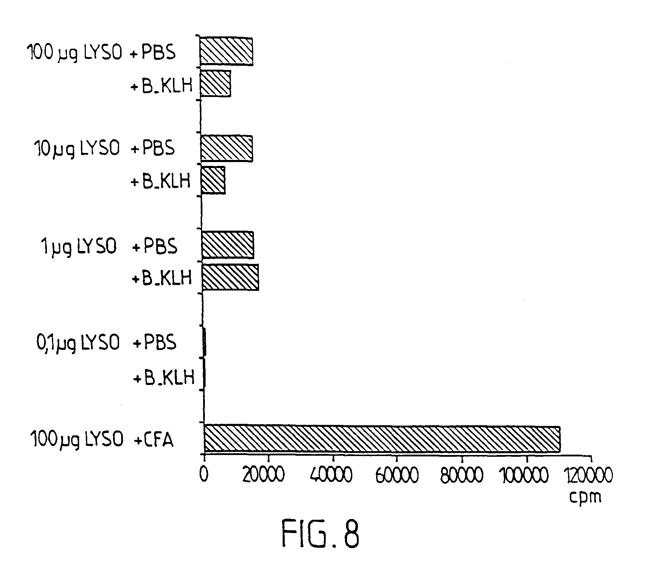

La figure 8 représente la réponse proliférative des cellules de souris immunisées par le lysozyme en adjuvant complet de Freund (CFA) ou en PBS avec des microparticules couplées au LH. L'addition de billes LH au lysozyme ne permet pas d'induire des réponses prolifératives élevées ce qui indique que le lysozyme doit être couplé de façon covalente aux microparticules pour induire des réponses T prolifératives.

### 2.5 - Induction de réponses T-prolifératives par injection d'hémoglobine ou d'ovalbumine couplée à des microparticules à des souris

Des souris ont été immunisées par l'hémoglobine ou l'ovalbumine en adjuvant complet de Freund, ou avec ces protéines couplées par liaison covalente au même type de particules que dans les exemples précédents (polystyrène, diamètre de l μm).

Les cellules des ganglions de ces animaux ont été restimulées in vitro par les protéines solubles et la prolifération cellulaire a été mesurée.

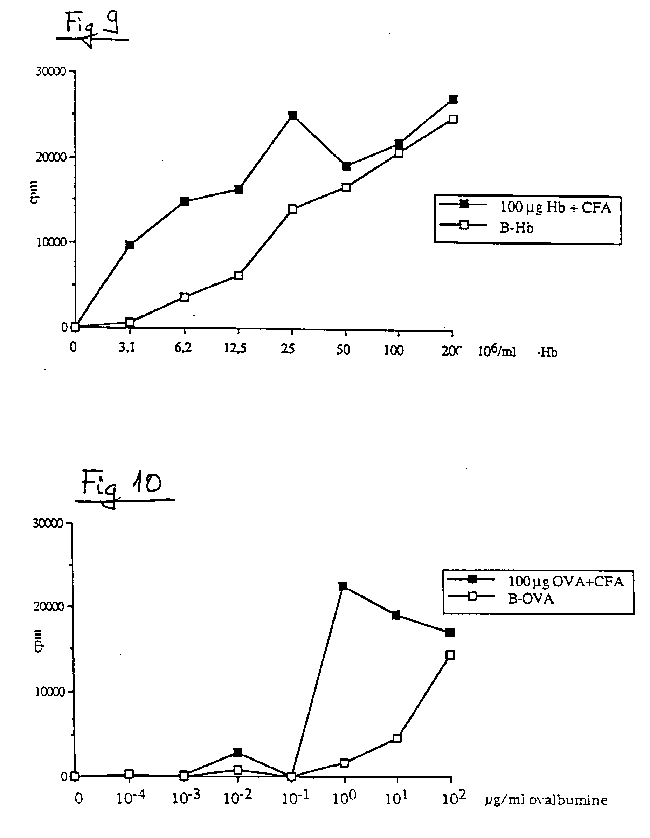

Les résultats obtenus pour l'hémoglobine (Hb) sont représentés sur la Figure 9, tandis que la Figure 10 illustre les résultats obtenus avec l'ovalbumine (OVA).

L'ensemble de ces résultats montre que ces protéines couplées à des microparticules sont capables de sensibiliser in vivo des lymphocytes T, CD4$^+$ spécifiques de ces protéines, en l'absence d'adjuvant.

### 2.6 - Induction de réponses T-prolifératives par injection de peptides synthétiques

#### 2.6.1 - Epitope T de la région C3 de la protéine VP1

L'épitope T de la région C3 (C3: T, 103-115) de la protéine du polyovirus a été synthétisé et couplé de façon covalente à des billes de l μm. Ces billes ont été injectées à des souris BALB/C.

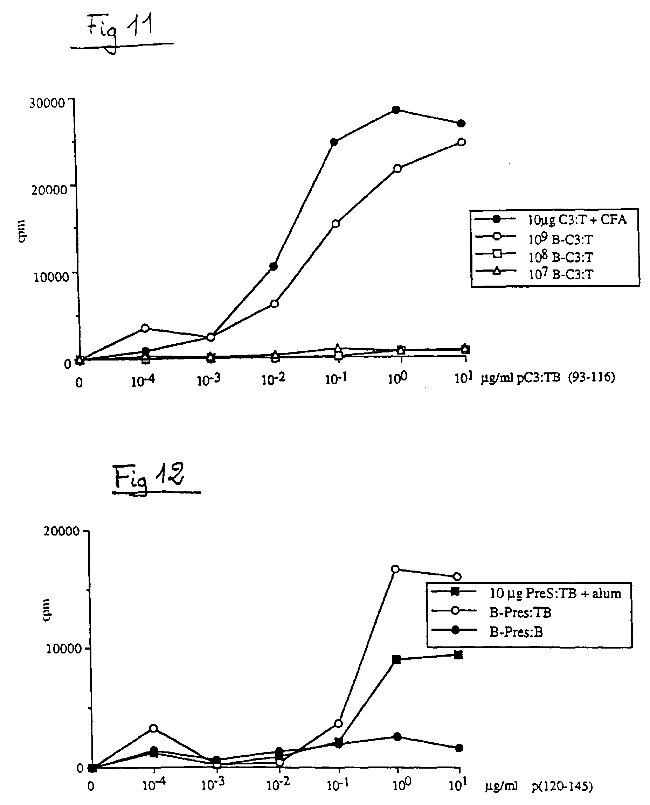

Les résultats de la Figure 11 établissent de manière claire que l'épitope T couplé aux billes (B-C3:T) induit une forte réponse T-proliférative pour des quantités de l'ordre de 10$^9$ billes injectées par souris.

#### 2.6.2 - Peptide pré-S:T de l'antigène HBS

Le peptide pré-S:T (120-132) de l'antigène HBS a été synthétisé et couplé de manière covalente par le glutaraldéhyde à des billes ayant un diamètre de l μm.

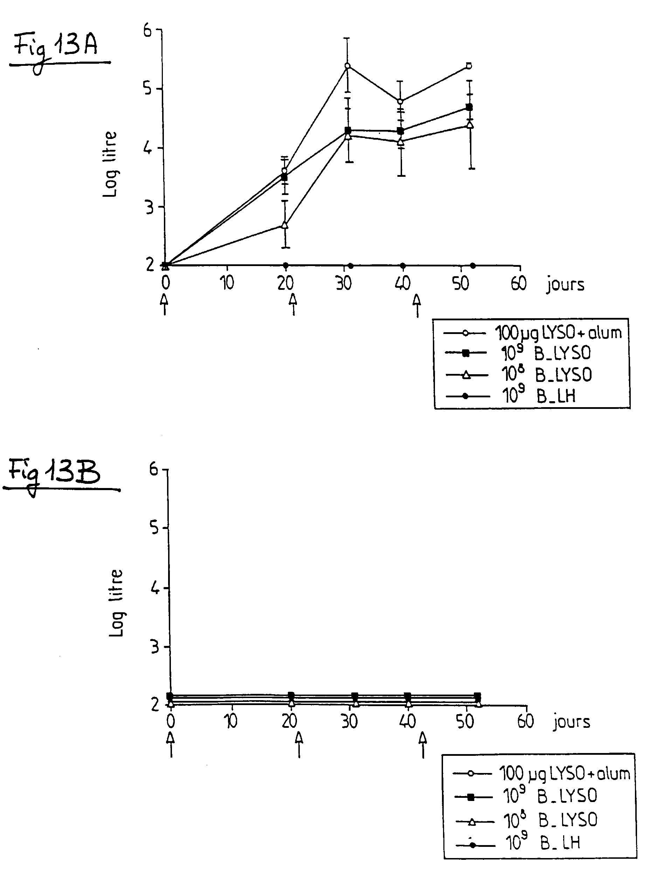

La Figure 12 montre que l'injection de 10$^9$ billes à des souris DBA/1 induit une forte réponse T-proliférative supérieure à celle obtenue avec le peptide on CFA. L'injection de billes ne contenant que l'épitope B n'induit pas de réponse proliférative, ce qui démontre la spécificité de la réponse.

### EXEMPLE 3.

### Induction de réponse anticorps par des microparticules portant un antigène.

Les matériels et méthodes sont similaires à ceux de l'Exemple 2.

### 1. Lysozyme et hémocyanine de Limulus

Pour les Figures 13A et 13B, des souris BALB/c ont été immunisées par injection intra-péritonéale avec 100 μg de lysozyme soluble on adjuvant (alum) ou avec les billes couplées à l'antigène: lysozyme ou Hémocyanine de Limulus (LH), sans aucun adjuvant.

Les injections ont été faites à JO, J21, J42, les sérums ont été prélevés à J20, J31, J40 et J52 ; et testés on ELISA pour leur teneur en anticorps. Les résultats sont exprimés on log10 du titre on anticorps anti-lysozyme (Figure 13A) et anti-KLH (Figure 13B).

Trois injections d'antigène ont été faites i.p. aux jours 0, 21 et 42. Les microparticules-lysozyme donnent de très bonnes réponses anticorps alors qu'aucune réponse anticorps n'est induite par les microparticules LH. Ces microparticules, par ailleurs, stimulent de façon très efficace les réponses T.

Une des différences entre le LH et le lysozyme réside dans leurs poids moléculaire (14500 pour lysozyme et 71000 pour LH).

A concentrations d'antigène couplé égales, la densité des molécules de LH sur les billes est donc environ 5 fois plus faible. Ceci pourrait expliquer l'absence de stimulation des réponses anticorps si celles-ci sont dues à la stimulation directe T-indépendante par l'antigène présent à forte densité sur les microparticules.

### 2. Hémoglobine et ovalbumine

Des souris ont été immunisées avec l'antigène soluble en adjuvant alum ou avec le même antigène sous forme particulaire, en l'absence d'adjuvant. L'apparition des anticorps a été alors suivie sur plusieurs semaines.

Dans le cas de l'hémoglobine (Hb), les souris ont été immunisées avec 100 μg de protéine ou 10$^9$ billes couplées à différentes densités avec la protéine (2.10$^4$ et 2.10$^5$ molécule/μm$^2$). Les billes portant l'ovalbumine (0VA) ont été testées à deux densités 7.10$^3$ et 7.10$^4$ molécule/μm$^2$).

Une première injection a été réalisée, puis deux autres injections ont été faites au vingt et unième jour et quarante deuxième jour. Les sérums ont été prélevés au vingtième jour, au trente et unième jour, au quarante et unième jour et au cinquante deuxième jour, puis ont été testés en ELISA pour leurs teneurs en anticorps IgG. Les résultats sont exprimés en Log du titre en anticorps.

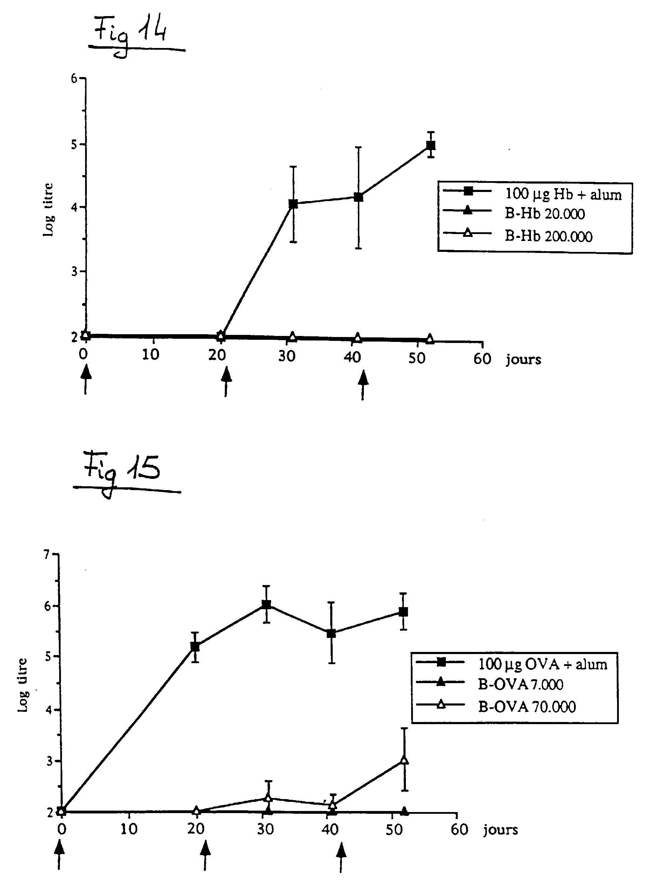

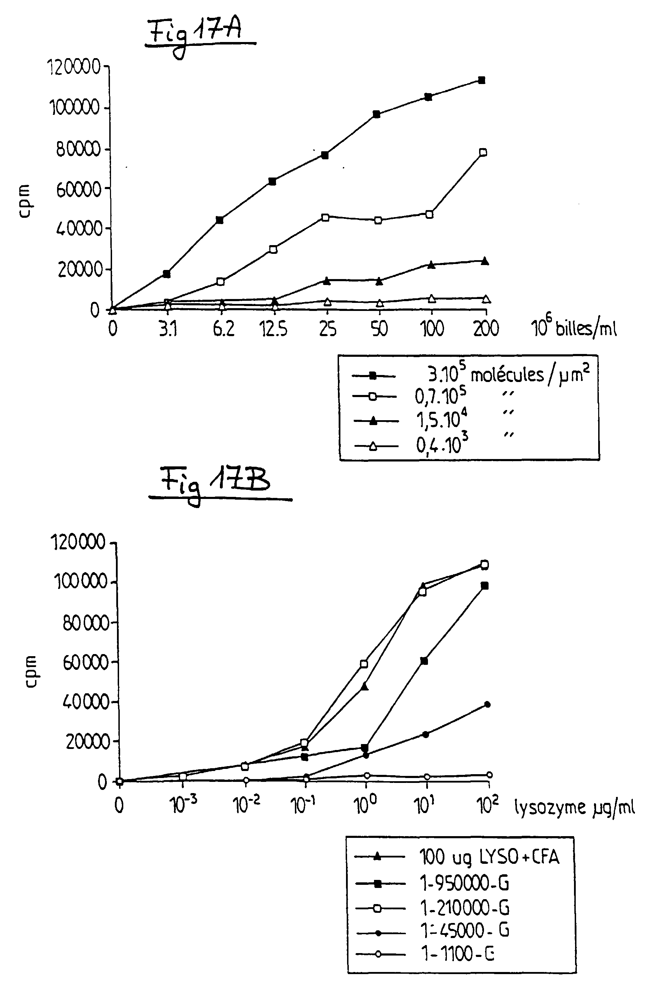

Les résultats de la Figure 14 montrent que l'hémoglobine couplé aux billes n'induit pas de réponse anticorps. Pour l'ovalbumine (Figure 15) des anticorps sont détectables après plusieurs injection si l'antigène est couplé à forte densité, mais ces réponses restent faibles. Ces résultats indiquent que des protéines de poids moléculaire élevé comme l'hémoglobine sont incapables d'induire des réponses anticorps, même si ces protéines sont couplées à une densité élevée sur des billes.

Ces résultats similaires à ceux obtenus avec le lysozyme et l'hémocyanine limulus confirment que des billes portant des protéines de haut poids moléculaire induisent des réponses T-prolifératives en l'absence de toute production d'anticorps.

De même, les protéines de poids moléculaire faible ou moyen, (inférieur à 50.000) peuvent induire l'apparition d'anticorps si elles sont couplées à de fortes densités aux billes.

### 3. Peptides synthétiques

Les peptides pré-S:TB (120-145) et pré-S:B correspondant à des parties de l'antigène HBS contenant respectivement un épitope T et un épitope B ou seulement l'épitope B ont été couplés de façon covalente à des billes de l $\mu$m par le glutaraldéhyde (B-pré-S:TB et B-pré-S:B).

La réponse anticorps induite par ces billes a été comparée à celle induite par 10 $\mu$g de peptide pré-S:TB soluble en adjuvant alum.

Les résultats de la Figure 16 montrent que les billes couplées au peptide TB, comprenant un épitope T et un épitope B induisent de fortes réponses anticorps, ce qui confirme que des antigènes de faibles poids moléculaires couplés à des billes permettent l'induction de réponse anticorps en l'absence de tout adjuvant. On notera que ces réponses sont aussi bonnes que celles obtenues avec le peptide libre en présence d'adjuvant alum.

### EXEMPLE 4

### Effet de la densité en lysozyme à la surface de microparticules sur leur immunogénéicité

Les matériels et méthodes utilisés sont similaires à ceux de l'Exemple 2.

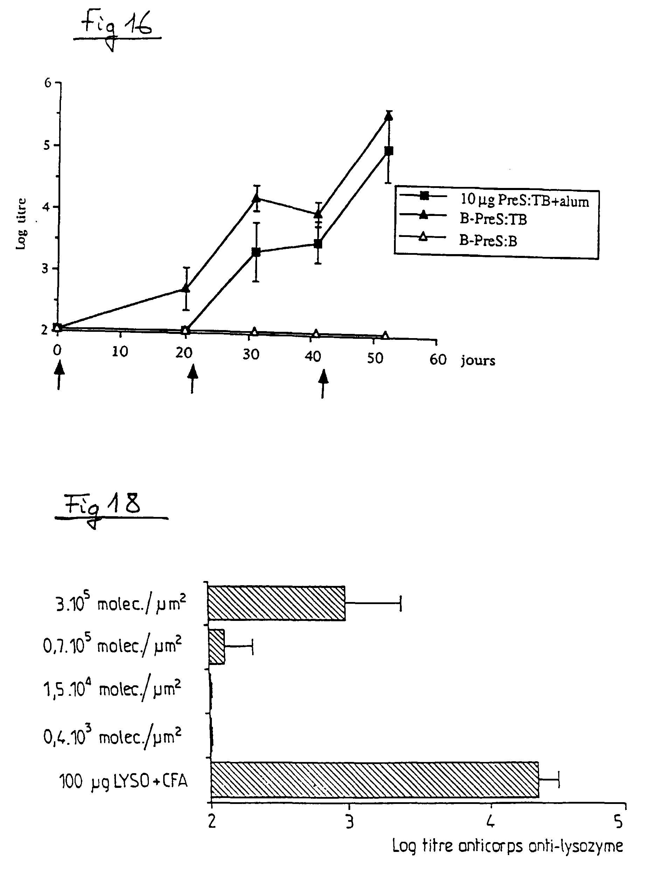

L'immunogénicité des billes couplées avec du lysozyme et présentant un nombre de molécules variables à leur surface a été testée dans les expériences présentées Figures 17 et 18.

Pour la Figure 17A, des souris BALB/c ont été immunisées par injection sous-cutanée avec 100 $\mu$g de lysozyme en CFA. Après 14 jours les ganglions inguinaux ont été prélevés et les cellules testées in vitro contre les billes portant différentes densités de lysozyme (de 1100 à 950.000 molécules de lysozyme ramenées à des billes de 1 $\mu$m de diamètre). Les résultats sont exprimés en cpm corrigés de la valeur obtenue sans antigène.

Pour la Figure 17B, des souris BALB/c ont été immunisées par injection sous-cutanée à la base de la queue avec du lysozyme soluble avec adjuvant (CFA) ou 10⁹ billes portant différentes densités de lysozyme sans aucun adjuvant.

Après 14 jours, les ganglions inguinaux ont été prélevés, et la réponse proliférative de ces cellules a été testée in vitro contre différentes concentrations de lysozyme ou billes. Les résultats sont exprimés en cpm corrigés de la valeur obtenue sans antigène.

La prolifération des cellules ganglionnaires provenant d'animaux immunisés par le lysozyme soluble en CFA a été testée après stimulation in vitro par les différentes microparticules-lysozyme. La réponse proliférative de ces cellules étant d'autant plus forte que la densité du lysozyme en surface des microparticules est élevée. Aucune prolifération des cellules ganglionnaires n'est obtenue après stimulation par les microparticules présentant une densité de 1.100 molécules de lysozyme par microparticule (figure 17A).

Dans l'expérience de la figure 17B, l'immunogénicité de ces microparticules a été testée in vivo. Des souris BALB/c ont été immunisées par les différentes microparticules, sans adjuvant, et les cellules ganglionnaires de ces animaux ont été stimulées in vitro par différentes concentrations de lysozyme soluble.

La prolifération des cellules ganglionnaires provenant d'animaux immunisés par les microparticules couplées au lysozyme à forte densité (95O.OOO et 210.000) est élevée, et comparable à la réponse des cellules sensibilisées par 100 $\mu$g de lysozyme en CFA. Après immunisation par les microparticules portant une densité moyenne de lysozyme (45.OOO), les cellules prolifèrent en réponse au lysozyme in vitro à partir de 10⁻¹ $\mu$g/ml. Les microparticules de plus faible densité n'ont pas sensibilisé les cellules T in vivo, car aucune prolifération n'a été observée en présence de lysozyme même à concentration élevée (figure 17B).

Il est à noter que 10⁹ microparticules couplées avec le lysozyme à haute densité correspondent à 23$\mu$g (1-950.000-G) et 5 $\mu$g (1-210.000-G) de lysozyme couplé, cependant la prolifération des cellules est aussi élevée qu'après injection de 100 $\mu$g de lysozyme en CFA.

Pour la Figure 18, des souris BALB/c ont été immunisées par injection sous-cutanée de lysozyme avec adjuvant (CFA) ou avec 10⁹ microparticules portant différentes densités de lysozyme (95O.OOO; 210.000, 45.OOO et 1100 molécules respectivement, ramenées à une microparticule de l $\mu$m de diamètre).

Après 14 jours, les sérums ont été prélevés et testés en ELISA pour leur teneur on anticorps anti-lysozyme. Les résultats sont exprimés en log10 du titre en anticorps.

La réponse humorale des souris immunisées par ces microparticules présentant différentes densités de lysozyme a été étudiée. L'injection de 100 $\mu$g de lysozyme en CFA induit un taux élevé d'anticorps anti-lysozyme (figure 18). Quatorze jours après l'immunisation, les billes couplées à la plus forte densité de lysozyme (95O.OOO) ont induit une production d'anticorps significative, alors que les billes de densité inférieure n'ont pas stimulé l'induction de réponse anticorps anti-lysozyme significative. Il faut noter, on particulier, que les billes de densité 210.000 qui ont induit une excellente prolifération spécifique du lysozyme n'ont pas stimulé la production d'anticorps.

Ces résultats révèlent que la prolifération de cellules T est induite avec des densités de lysozyme allant de 45.000 à 950.000 molécules de lysozyme par microparticule, alors que la production d'anticorps nécessite une densité importante de protéine couplée aux microparticules.

Au sens de la présente description, l'expression "microparticules " désigne des particules pouvant avoir diverses configurations géométriques et spatiales. Dans la pratique, il s'agit préférentiellement de microsphères ou billes, telles qu'elles sont obtenues par les techniques usuelles de fabrication des polymères.

## Revendications

1. Utilisation de microparticules, ayant un diamètre moyen compris entre environ 0,25 et environ 1,5 μm, en matériau synthétique polymère, portant en surface une ou plusieurs protéines ou peptides liés de manière covalente, la ou lesdits protéines ou peptides portant chacun un ou plusieurs épitopes, pour la fabrication d'un médicament ou d'un vaccin pour l'induction d'une réponse immunitaire, les densités de la ou des protéines ou peptides a la surface des microparticules étant ajustées afin d'orienter ladite réponse immunitaire, vers l'induction d'une réponse humorale et cellulaire ou vers l'induction d'une réponse majoritairement cellulaire.

2. Utilisation selon la revendication 1, pour l'induction de réponses cellulaire et/ou humorale, caractérisée en ce que les microparticules présentent une densité pour chacune des protéines ou peptides portant un ou plusieurs épitopes d'au minimum $10^5$ molécules/$\mu m^2$ et préférentiellement $5.10^5$ protéines/$\mu m^2$.

3. Utilisation selon la revendication I, pour l'induction d'une réponse majoritairement cellulaire, caractérisée en ce que les microparticules présentent une densité pour chacune des protéines ou peptides portant un ou peptides épitopes, comprise environ entre $IO^4$ et $5.10^4$ molécules/$\mu m^2$.

4. Utilisation selon l'une des revendications I à 3, caractérisée en ce que les microparticules ont un diamètre moyen de 1μm.

5. Utilisation selon l'une des revendications I à 4, caractérisée en ce que la liaison est effectuée par réaction des fonctions $NH_2$ et/ou CO des protéines et du matériau constituant la microparticule.

6. Utilisation selon l'une des revendications 1 à 4, caractérisée en ce que la liaison des protéines ou des peptides et du matériau constituant la microparticule est covalente et est effectuée avec ou sans agent pontant.

7. Utilisation selon la revendication 6, caractérisée en ce que l'agent pontant est le glutaraldéhyde ou le carbodiimide.

8. Utilisation selon l'une des revendications I à 7, caractérisée en ce que ledit matériau est un polymère biocompatible.

9. Utilisation selon la revendication 8, caractérisée en ce que ledit polymère est le polyacroléine ou le polystyrène ou des polymères d'acide lactique ou des copolymères d'acides lactique et glycolique.

10. Utilisation selon l'une des revendications 1 à 9, pour la fabrication d'un médicament pour la thérapeutique humaine, caractérisée en ce que ledit polymère est biodégradable.

11. Utilisation selon l'une des revendications I à 10 caractérisée en ce que les microparticules portent en surface des molécules susceptibles d'activer le système immunitaire.

12. Procédé pour la fabrication d'un médicament ou d'un vaccin dont la réponse immunitaire est soit majoritairement humorale soit majoritairement cellulaire, ledit procédé étant caractérisé en ce qu'on fixe de manière covalente sur des microparticules, ayant un diamètre moyen compris entre environ 0,25 et environ 1,5 μm, en matériau synthétique polymère au moins une protéine ou un peptide portant un ou plusieurs épitopes ou des peptides contenant uniquement des épitopes T ou B ou une composition des deux en faisant varier la densité de la protéine ou du peptide fixé à la surface selon le type de réponse désirée.

13. Procédé selon la revendication 12, caractérisé en ce qu'on utilise des microparticules comme indiqué à l'une quelconque des revendications I à 11.

14. Microparticule, ayant un diamètre moyen compris entre environ 0,25 et environ 1,5 μm, en un matériau synthétique polymère portant en surface une ou plusieurs protéines ou peptides liés de manière covalente au matériau constituant la microparticule, la ou lesdites protéines ou peptides portant chacune un ou plusieurs épitopes, et étant présentes à des densités comprises entre $IO^4$ et $5.IO^5$ protéines/$\mu m^2$ pour chacune des protéines.

15. Microparticule selon la revendication 14, caractérisée en ce qu'elle a un diamètre moyen de 1μm.

**16.** Microparticule selon l'une des revendications 14 et 15, caractérisée en ce que la liaison est effectuée par réaction des fonctions $NH_2$ et/ou CO des protéines et du matériau constituant la microparticule.

**17.** Microparticule selon l'une des revendications 14 et 15, caractérisée en ce que la liaison des protéines ou peptides et du matériau constituant la microparticule est effectuée par l'intermédiaire d'un agent pontant.

**18.** Microparticule selon la revendication 17, caractérisée en ce que l'agent pontant est le glutaraldéhyde, ou le carbodiimide.

**19.** Microparticule selon l'une des revendications 14 à 18, caractérisée en ce qu'elle est composée d'un polymère biocompatible.

**20.** Microparticule selon la revendication 19, caractérisée en ce que ledit polymère est la poly(acroléine) ou le polystyrène, un polymère d'acide lactique ou un copolymère d'acide lactique et glycolique.

**21.** Microparticule selon la revendication 19, pour l'application en thérapeutique humaine, caractérisée en ce que ledit polymère est biodégradable.

**22.** Microparticule selon l'une des revendications 14 à 21, caractérisée en ce qu'elle porte en surface des molécules susceptibles d'activer le système immunitaire.

**23.** Microparticule selon l'une des revendications 14 à 22, caractérisée en ce que ladite protéine ou peptides comprend l'épitope B de la région pré-$s_2$ de l'antigène HBs du virus de l'hépatite virale.

**24.** Microparticule selon l'une des revendications 14 à 22, caractérisée en ce que ladite protéine comprend l'épitope B de la protéine VP1 du virus de la poliomyélite.

**25.** Microparticule selon l'une des revendications 14 à 22, caractérisée en ce que ladite protéine ou peptides comprend l'épitope B de la protéine gp 12O du virus HIV-1.

**26.** Médicament ou vaccin, caractérisé en ce qu'il comprend des microparticules selon l'une des revendications 14 à 25.

**27.** Composition pharmaceutique caractérisée en ce qu'elle comprend des microparticules selon l'une des revendications 14 à 25 en association avec des diluants et adjuvants pharmaceutiquement compatibles.

## Claims

**1.** Use of synthetic polymer microparticles having an average diameter of between about 0.25 μm and about 1.5 μm, having on their surface one or more covalently bonded proteins or peptides, said protein(s) or peptide(s) each having one or more epitopes, for the production of a drug or vaccine for inducing an immune response, the densities of the protein(s) or peptide(s) on the microparticle surfaces being ajusted so as to direct said immune response towards the induction of a humoral and cellular response or towards the induction of a largely cellular response.

**2.** Use according to claim 1, for the induction of cellular and/or humoral responses, characterized in that the microparticles have a density for each of the proteins or peptides having one or several epitopes of a minimum of $10^5$ molecules/$\mu m^2$ and preferably $5.10^5$ molecules/$\mu m^2$.

**3.** Use according to claim 1, for the induction of a largely cellular response, characterized in that the microparticles have a density for each of the proteins or peptides having one or several epitopes of between $10^4$ and $5.10^4$ molecules/$\mu m^2$.

**4.** Use according to one of claims 1 to 3, characterized in that the microparticles have an average diameter of 1 μm.

**5.** Use according to one of claims 1 to 4, characterized in that the bond is made by reaction between the $NH_2$ and/or CO groups of the proteins and the material making up the microparticle.

**6.** Use according to one of claims 1 to 4, characterized in that the bond between the proteins or the peptides and the material making up the microparticle is covalent and made with or without a bridging reagent.

**7.** Use according to claim 6, characterized in that the bridging reagent is glutaraldehyde or carbodiimide.

**8.** Use according to one of claims 1 to 7, characterized in that said material is a biocompatible polymer.

**9.** Use according to claim 8, characterized in that said polymer is polyacrolein or polystyrene or lactic acid polymers or copolymers of lactic and glycolic acids.

**10.** Use according to one of claims 1 to 9, for the production of a drug for human therapeutics, characterized in that said polymer is biodegradable.

**11.** Use according to one of claims 1 to 10, characterized in that the microparticles have on their surface molecules liable to activate the immune system.

**12.** Process for the production of a drug or vaccine whose immune response is either largely humoral or largely cellular, said process being characterized in that at least one protein or peptide having one or more epitopes or peptides containing T or B epitopes only or a combination of the two are covalently fixed to synthetic polymer microparticles having an average diameter of between about 0.25 µm and about 1.5 µm, the density of the protein or the peptide fixed to the surface being varied according to the type of response required.

**13.** Process according to claim 12, characterized in that microparticles as defined in any one of claims 1 to 12 are used.

**14.** Synthetic polymer microparticle having an average diameter of between about 0,25 µm and about 1,5 µm, having on its surface one or more proteins or peptides covalently bonded to the material of the microparticle, said protein(s) or peptide(s) each having one or more epitopes, and being provided at a density of between $10^4$ and $5.10^5$ molecules/µm$^2$ for each of the proteins.

**15.** Microparticle according to claim 14, characterized in that it has an average diameter of 1 µm.

**16.** Microparticle according to one of claims 14 and 15, characterized in that the bond is made by reaction between the $NH_2$ and/or CO groups of the proteins and the material making up the microparticle.

**17.** Microparticle according to one of claims 14 and 15, characterized in that the bond between the proteins or the peptides and the material making up the microparticle is made by use of a bridging reagent.

**18.** Microparticle according to claim 17, characterized in that the bridging reagent is glutaraldehyde, or carbodiimide.

**19.** Microparticle according to one of claims 14 to 18, characterized in that it is composed of a biocompatible polymer.

**20.** Microparticle according to claim 19, characterized in that said polymer is poly(acrolein) or polystyrene, a lactic acid polymer or a copolymer of lactic and glycolic acids.

**21.** Microparticle according to claim 19, for application in human therapeutics, characterized in that said polymer is biodegradable.

**22.** Microparticle according to one of claims 14 to 21, characterized in that it has on its surface molecules liable to activate the immune system.

**23.** Microparticle according to one of claims 14 to 22, characterized in that said protein or said peptide comprises the B epitope from the pre-s$_2$ region of the HBs antigen of the viral hepatitis virus.

**24.** Microparticle according to one of claims 14 to 22, characterized in that said protein or said peptide comprises the B epitope of the VP1 protein of the poliomyelitis virus.

**25.** Microparticle according to one of claims 14 to 22, characterized in that said protein or said peptide comprises the B epitope of the gp 120 protein of the HIV-1 virus.

**26.** Drug or vaccine, characterized in that it comprises microparticles according to one of claims 14 to 25.

**27.** Pharmaceutical composition characterized in that it comprises microparticles according to one of claims 14 to 25, in combination with pharmaceutically compatible diluents or vehicles.

**Patentansprüche**

**1.** Verwendung von Mikroteilchen mit einem mittleren Durchmesser zwischen etwa 0,25 und etwa 1,5 µm aus synthetischem polymerem Material, das auf der Oberfläche ein oder mehrere kovalent gebundene Proteine oder Peptide trägt, wobei das Protein oder Peptid bzw. jedes der Proteine oder Peptide ein oder mehrere Epitope trägt, zur Herstellung eines Medikaments oder eines Impfstoffs für die Induktion einer Immunantwort, wobei die Dichte des Proteins oder Peptids oder der Proteine oder Peptide auf der Oberfläche der Mikroteilchen so eingestellt wird, dass die Immunantwort in die Richtung der Induktion einer humoralen und zellulären Antwort oder in die Richtung der Induktion einer hauptsächlich zellulären Antwort gelenkt wird.

**2.** Verwendung gemäß Anspruch 1 zur Induktion zellulärer und/oder humoraler Antworten, dadurch gekennzeichnet, dass die Mikroteilchen für jedes der Proteine oder Peptide, die ein oder mehrere Epitope tragen, eine Dichte von wenigstens $10^5$ Molekülen/µm$^2$ und vorzugsweise $5 \cdot 10^5$ Proteinen/µm$^2$ aufweisen.

**3.** Verwendung gemäß Anspruch 1 zur Induktion einer hauptsächlich zellulären Antwort, dadurch gekennzeichnet, dass die Mikroteilchen für jedes der Proteine oder Peptide, die ein oder mehrere Epitope tragen, eine Dichte zwischen etwa $10^4$ und $5 \cdot 10^4$

Molekülen/$\mu m^2$ aufweisen.

**4.** Verwendung gemäß einem der Ansprüche 1 bis 3, dadurch gekennzeichnet, dass die Mikroteilchen einen mittleren Durchmesser von 1 $\mu m$ haben.

**5.** Verwendung gemäß einem der Ansprüche 1 bis 4, dadurch gekennzeichnet, dass die Bindung durch eine Reaktion der $NH_2$- und/oder CO-Funktionen der Proteine und des Materials, aus dem das Mikroteilchen besteht, erfolgt.

**6.** Verwendung gemäß einem der Ansprüche 1 bis 4, dadurch gekennzeichnet, dass die Bindung zwischen den Proteinen oder Peptiden und dem Material, aus dem das Mikroteilchen besteht, kovalent ist und mit oder ohne Kupplungsmittel gebildet wird.

**7.** Verwendung gemäß Anspruch 6, dadurch gekennzeichnet, dass es sich bei dem Kupplungsmittel um Glutaraldehyd oder Carbodiimid handelt.

**8.** Verwendung gemäß einem der Ansprüche 1 bis 7, dadurch gekennzeichnet, dass das Material ein biokompatibles Polymer ist.

**9.** Verwendung gemäß Anspruch 8, dadurch gekennzeichnet, dass es sich bei dem Polymer um Polyacrolein oder Polystyrol oder Milchsäurepolymere oder Milchsäure-Glycolsäure-Copolymere handelt.

**10.** Verwendung gemäß einem der Ansprüche 1 bis 9 zur Herstellung eines Medikaments für die Humantherapie, dadurch gekennzeichnet, dass das Polymer biologisch abbaubar ist.

**11.** Verwendung gemäß einem der Ansprüche 1 bis 10, dadurch gekennzeichnet, dass die Mikroteilchen auf der Oberfläche Moleküle tragen, die das Immunsystem aktivieren können.

**12.** Verfahren zur Herstellung eines Medikaments oder eines Impfstoffs, dessen Immunantwort entweder hauptsächlich humoral oder hauptsächlich zellulär ist, wobei das Verfahren dadurch gekennzeichnet ist, dass man wenigstens ein Protein oder Peptid, das ein oder mehrere Epitope trägt, oder Peptide, die ausschließlich T- oder B-Epitope enthalten, oder eine Kombination von beiden kovalent auf Mikroteilchen mit einem mittleren Durchmesser zwischen etwa 0,25 und etwa 1,5 $\mu m$ aus synthetischem polymerem Material fixiert, indem man die Dichte des auf der Oberfläche fixierten Proteins oder Peptids je nach der Art der gewünschten Antwort variiert.

**13.** Verfahren gemäß Anspruch 12, dadurch gekennzeichnet, dass man Mikroteilchen verwendet, wie sie in einem der Ansprüche 1 bis 12 angegeben sind.

**14.** Mikroteilchen mit einem mittleren Durchmesser zwischen etwa 0,25 und 1,5 $\mu m$ aus synthetischem polymerem Material, das auf der Oberfläche ein oder mehrere Proteine oder Peptide trägt, die kovalent an das Material gebunden sind, aus dem das Mikroteilchen besteht, wobei das Protein oder Peptid bzw. jedes der Proteine oder Peptide ein oder mehrere Epitope trägt und jedes der Proteine in einer Dichte zwischen $10^4$ und $5 \cdot 10^5$ Proteinen/$\mu m^2$ vorhanden ist.

**15.** Mikroteilchen gemäß Anspruch 14, dadurch gekennzeichnet, dass es einen mittleren Durchmesser von 1 $\mu m$ hat.

**16.** Mikroteilchen gemäß einem der Ansprüche 14 und 15, dadurch gekennzeichnet, dass die Bindung durch eine Reaktion der $NH_2$-und/oder CO-Funktionen der Proteine und des Materials, aus dem das Mikroteilchen besteht, erfolgt.

**17.** Mikroteilchen gemäß einem der Ansprüche 14 und 15, dadurch gekennzeichnet, dass die Bindung zwischen den Proteinen oder Peptiden und dem Material, aus dem das Mikroteilchen besteht, mit Hilfe eines Kupplungsmittels gebildet wird.

**18.** Mikroteilchen gemäß Anspruch 17, dadurch gekennzeichnet, dass es sich bei dem Kupplungsmittel um Glutaraldehyd oder Carbodiimid handelt.

**19.** Mikroteilchen gemäß einem der Ansprüche 14 bis 18, dadurch gekennzeichnet, dass es aus einem biokompatiblen Polymer besteht.

**20.** Mikroteilchen gemäß Anspruch 19, dadurch gekennzeichnet, dass es sich bei dem Polymer um Polyacrolein oder Polystyrol, ein Milchsäurepolymer oder ein Copolymer von Milchsäure und Glycolsäure handelt.

**21.** Mikroteilchen gemäß Anspruch 19 zur Anwendung in der Humantherapie, dadurch gekennzeichnet, dass das Polymer biologisch abbaubar ist.

**22.** Mikroteilchen gemäß einem der Ansprüche 14 bis 21, dadurch gekennzeichnet, dass es auf der Oberfläche Moleküle trägt, die das Immunsystem aktivieren können.

**23.** Mikroteilchen gemäß einem der Ansprüche 14 bis 22, dadurch gekennzeichnet, dass das Protein oder Peptid das B-Epitop des $Präs_2$-Bereichs des Antigens HBs des Hepatitis-A-Virus umfasst.

**24.** Mikroteilchen gemäß einem der Ansprüche 14 bis 22, dadurch gekennzeichnet, dass das Protein oder Peptid das B-Epitop des Proteins VP1 des Poliomyelitis-Virus umfasst.

**25.** Mikroteilchen gemäß einem der Ansprüche 14 bis 22, dadurch gekennzeichnet, dass das Protein oder Peptid das B-Epitop des Proteins gp120 des Virus HIV-1 umfasst.

**26.** Medikament oder Impfstoff, dadurch gekennzeichnet, dass es bzw. er Mikroteilchen gemäß einem der Ansprüche 14 bis 25 umfasst.

**27.** Pharmazeutische Zusammensetzung, dadurch gekennzeichnet, dass sie Mikroteilchen gemäß einem der Ansprüche 14 bis 25 zusammen mit pharmazeutisch annehmbaren Verdünnungsmitteln und Adjuvantien umfasst.

FIG.1

FIG.2A

KLH

cpm

—o— cellules de rate
—△— macrophages
—□— cellules B activées
      par le LPS

Concentration en antigène (μg/ml)

FIG.2B

TNP-KLH

cpm

Concentration en antigène (μg/ml)

FIG.2C

B(KLH)

cpm

Concentration en antigène (10$^6$/ml)

FIG.2D

B(TNP-KLH)

cpm

Concentration en antigène (10$^6$/ml)

EP 0 662 000 B1

# FIG. 3A

# FIG. 3B

# FIG. 4A

# FIG.4B

FIG.5A

FIG.5 B

## FIG.6A

cpm

0    50000    100000    150000

$10^{-3}$  $10^{-2}$  $10^{-1}$  $10^0$  $10^1$  $10^2$

lysozyme µg/ml

| | |
|---|---|
| —●— | 100 µg LYSO + ACF |
| —■— | $10^9$ B-LYSO |

## FIG.6B

cpm

0    20000    40000    60000

$10^{-3}$  $10^{-2}$  $10^{-1}$  $10^0$  $10^1$  $10^2$

lysozyme µg/ml

| | |
|---|---|
| —■— | $10^9$ B_LYSO |
| —□— | $10^8$ B_LYSO |
| —▲— | $10^9$ B_KLH |
| —△— | $10^8$ B_KLH |

## FIG. 7A

cpm

125000
100000
75000
50000
25000
0

0    $10^{-4}$    $10^{-3}$    $10^{-2}$    $10^{-1}$    $10^0$    $10^1$    $10^2$

lysozyme µg/ml

- ●— 100 µg LYSO+CFA
- ■— 10 µg LYSO+CFA
- ▲— 1 µg LYSO+CFA
- ◆— 0,1 µg LYSO+CFA
- ✕— 0,01 µg LYSO+CFA

## FIG. 7B

cpm

100000
80000
60000
40000
20000
0

0    $10^{-4}$    $10^{-3}$    $10^{-2}$    $10^{-1}$    $10^0$    $10^1$    $10^2$

lysozyme µg/ml

- ■— $10^9$ B-LYSO
- ●— $10^8$ B-LYSO
- ▲— $10^7$ B-LYSO
- ◆— $10^6$ B-LYSO

FIG.8

## Fig 9

## Fig 10

Fig 11

Fig 12

Fig 13A

Fig 13B

_Fig 14_

_Fig 15_

Fig 17A

Fig 17B

Fig 16

Fig 18